# EUROPEAN PATENT SPECIFICATION

(11) **EP 3 740 755 B1**
(45) Date of publication and mention of the grant of the patent: **07.06.2023**
(21) Application number: 19700508.5
(22) Date of filing: 15.01.2019
(51) Int. Cl.: G01N 33/574, A61K 31/00

(54) **USE OF THE TAS1R3 PROTEIN AS A MARKER FOR THERAPEUTIC, DIAGNOSTIC, AND/OR PROGNOSTIC PURPOSES FOR TUMORS THAT EXPRESS SAID PROTEIN**
VERWENDUNG DES TAS1R3-PROTEINS ALS MARKER FÜR THERAPEUTISCHE, DIAGNOSTISCHE UND/ODER PROGNOSTISCHE ZWECKE FÜR TUMORE MIT EXPRESSION DIESES PROTEINS
UTILISATION DE LA PROTÉINE TAS1R3 COMME MARQUEUR À DES FINS THÉRAPEUTIQUES, DIAGNOSTIQUES, ET/OU À DES FINS DE PRONOSTIC POUR DES TUMEURS EXPRIMANT CETTE PROTÉINE

(30) Priority: 15.01.2018 EP 18382013
(43) Date of publication of application: 25.11.2020
(73) Proprietor: Fundación Instituto de Investigación Sanitaria de Santiago de Compostela (FIDIS), Santiago de Compostela (ES); Servizo Galego De Saúde, 15703 Santiago de Compostela (ES)
(72) Inventor: DE LA FUENTE FREIRE, María, 15706 Santiago de Compostela (ES); LÓPEZ LÓPEZ , Rafael, 15706 Santiago de Compostela (ES); ALONSO NOCELO, Marta, 15706 Santiago de Compostela (ES); VÁZQUEZ RÍOS, Abi Judit, 15706 Santiago de Compostela (ES)
(74) Representative: Hoffmann Eitle
(86) International application number: PCT/EP2019/050981
(87) International publication number: WO 2019/138140

(56) References cited:
- EP-A1- 3 067 698
- US-A1- 2003 040 045
- ERIC M. WAUSON ET AL: "Amino acid regulation of autophagy through the GPCR TAS1R1-TAS1R3", AUTOPHAGY, vol. 9, no. 3, 7 March 2013 (2013-03-07), pages 418-419, XP055477394, US ISSN: 1554-8627, DOI: 10.4161/auto.22911
- TOYONO ET AL: "CCAAT/Enhancer-binding protein beta regulates expression of human T1R3 taste receptor gene in the bile duct carcinoma cell line, HuCCT1", BIOCHIMICA ET BIOPHYSICA ACTA . GENE STRUCTURE AND EXPRESSION, ELSEVIER, AMSTERDAM, NL, vol. 1769, no. 11-12, 1 November 2007 (2007-11-01), pages 641-648, XP022349465, ISSN: 0167-4781

## Description

### Technical field.

The invention presents the use of the TAS1R3 protein as a new marker and molecular target in epithelial tumors and other human pathologies. In particular, the use of the detection of the TAS1 R3 protein as well as its expression products, as a marker for therapeutic, diagnostic or prognostic purposes, for tumors that normally express said protein is part of the present invention.

### Background of the invention.

Significant progress has been made in the chemotherapy of neoplastic diseases over the past 30 years. This includes some progress in the development of new chemotherapeutic agents, and more particularly, in the development of regimens for the concurrent administration of drugs. A significant understanding of the neoplastic processes at the cellular and tissue level, and the mechanism of action of the basic antineoplastic agents, has also made it possible to obtain advances in the chemotherapy of a number of neoplastic diseases, including choriocarcinoma, Wilm's tumor, acute leukemia, rhabdomyosarcoma, retinoblastoma, Hodgkin's disease, and Burkitt's lymphoma. However, despite the progress made so far in the field of oncology, and in particular in chemotherapy, many of the most prevalent forms of human cancer are still resistant to the current chemotherapeutic treatments.

In this sense, in any oncological treatment regimen and for it to be effective, the concept of "total cell annihilation" is crucial. This concept holds that, in order to have an effective treatment regimen, either through a surgical or chemotherapeutic approach, or both, there must be a total cellular annihilation of all so-called "clonogenic" malignant cells, that is, of the cells that have the capacity to grow in an uncontrolled way, and to replace any tumor mass that could be eliminated. Due to the ultimate need to develop therapeutic agents and regimens that achieve total cell elimination, certain types of tumors have been more susceptible than others to therapy. For example, soft tissue tumors (e.g., lymphomas), and tumors of blood and blood-forming organs (e.g., leukemia) have generally responded more satisfactorily to chemotherapeutic therapy than solid tumors such as carcinomas. One reason for the susceptibility of soft tumors to chemotherapy is the greater physical accessibility to the lymphoma and leukemia cells in the chemotherapeutic intervention. Simply put, it is much more difficult for most chemotherapeutic agents to reach all the cells of a solid tumor mass, than to reach the soft tumors, and therefore, it is much more difficult to achieve total cell destruction in the case of solid tumors. In this regard, to treat such tumors, solid tumors, the doses of chemotherapeutic agents are significantly increased resulting in side effects, which generally limits the overall effectiveness of the therapy.

The strategy to develop satisfactory antitumor agents capable of treating solid tumors, therefore, involves the design of agents that selectively kill tumor cells, while exerting relatively few, if any, adverse effects against normal tissues. This objective has been difficult to achieve, because there are few qualitative differences between neoplastic and normal tissues. Due to this, in recent years, many efforts have been devoted to trying to identify tumor specific "marker antigens", which can serve as immunological targets, both for chemotherapy and for diagnosis. In this sense, the present invention addresses this aspect by providing a new biomarker that is differentially expressed in the cell membrane of tumor cells in both primary tumors and disseminated cells. Additionally, the expression of this biomarker is related to a greater invasive and tumorigenic capacity of the tumor cells, increasing this expression especially under conditions of absence of nutrients, being able, therefore, to use this biomarker for the diagnosis/prognosis of cancer. Finally, since it is a membrane receptor, it is possible to design targeted therapies, for example ligands such as small molecules, peptides or antagonists, that stop cell proliferation and the ability of cells to migrate, invade and disseminate. So, in the past, immunotoxins have been used to selectively target cancer cells of solid tumors. Immunotoxins are conjugates of a specific target agent, typically an antibody or fragment thereof directed to the tumor, with a cytotoxic agent, such as a toxin moiety. The target agent is designed to direct the toxin to the cells that carry the target antigen, and kill such cells. On the other hand, "second generation" immunotoxins, such as those that use deglycosylated ricin A chain, have been developed to prevent hepatic entrapment of the immunotoxin and reduce hepatotoxicity (Blakey et al., 1987a; b), and those with new crosslinks to provide the immunotoxins with a higher *in vivo* stability (Thorpe et al., 1988). Immunotoxins have been shown to be effective in the treatment of lymphomas and leukemia in mice (Thorpe et al., 1988, Ghetie et al., 1991, Griffin et al., 1988a; b) as well as in humans (Vitetta et al., 1991). The present invention therefore provides specific target agents against the biomarker of the present invention, useful for the synthesis of immunotoxins.

In addition, the identification of new biomarkers, such as the one detailed in the present invention, are extremely useful for the development of personalized medicine, both for diagnostic and prognostic purposes, as well as for the selection of targeted therapies, and monitoring of the treatment. Personalized medicine is based on the premise that each tumor is unique, and undergoes dynamic changes over time, so it is important to have tools that allow determining the best therapeutic approach at all times. In the field of personalized medicine, liquid biopsy stands out for its determining role. Today, we know that tumors originate due to the appearance of genetic alterations that affect the proliferation and survival mechanisms of cells, as well as being complex and heterogeneous in their molecular composition. On the other hand, once the cancer is diagnosed, the primary tumor, or its metastases, must be characterized by analyzing the DNA, RNA and protein profile. Within the same tumor different tumor clones coexist that mark the evolution of the tumor and the resistance to the various therapies, hence, the term "dynamic tumors" has been coined. These are susceptible to variations over time, the determination of the dominant clone at each moment being key to select the most effective therapeutic strategy. The diagnosis of non-small-cell lung cancer (NSCLC) is usually performed by CT scan, a sensitive but not specific technique, which requires additional follow-up through invasive procedures (biopsy), which is why clinical practice requires new biomarkers that provide information attached to diagnostic imaging, as well as biomarkers that are able to provide information without the need to apply such aggressive procedures as a biopsy.

### Brief description of the invention

In the present invention, the use of the TAS1R3 receptor as a biomarker for application in cancer diagnosis, monitoring, and therapy is described for the first time. In this sense, the authors of the present invention have demonstrated that TAS1 R3 is a biomarker of interest in oncology, useful for the diagnosis of the disease, and capable of providing relevant information thereupon, to monitor the evolution, select the treatment, and selectively direct therapeutic molecules. It has been determined that it is possible to identify therapies against this receptor, and that it is also possible to direct conjugates and controlled drug-release systems, such as nanoparticles, very efficiently observing an intracellular accumulation thereof in primary, disseminated and metastatic tumor cells. On the other hand, the presence of TAS1R3 in circulating tumor cells (CTCs) has also been demonstrated. These are tumor cells released into the bloodstream by the primary tumor and are considered key factors in the creation of metastases, so that their detection in early stages will serve as an early detector of metastasis, and are also useful in monitoring the disease and evaluating the response to drugs.

Overall, this discovery opens the doors to the functionalization of any type of molecule with a compound that acts as a ligand against the TAS1R3 receptor, preferably a ligand selected from the group consisting of antibodies, antibody fragments, aptamers, peptides, or low-molecular-weight hydrophobic or hydrophilic molecules, such as lactisole, as well as ligand-drug or ligand-radioisotope conjugates, capable of binding to the TAS1R3 receptor, or ligands functionalized with reactive groups, for use as pharmacological and/or diagnostic vehicles against tumor cells, in particular against CTCs (circulating tumor cells) and against primary, disseminated, or metastatic tumor cells.

### Brief description of the figures

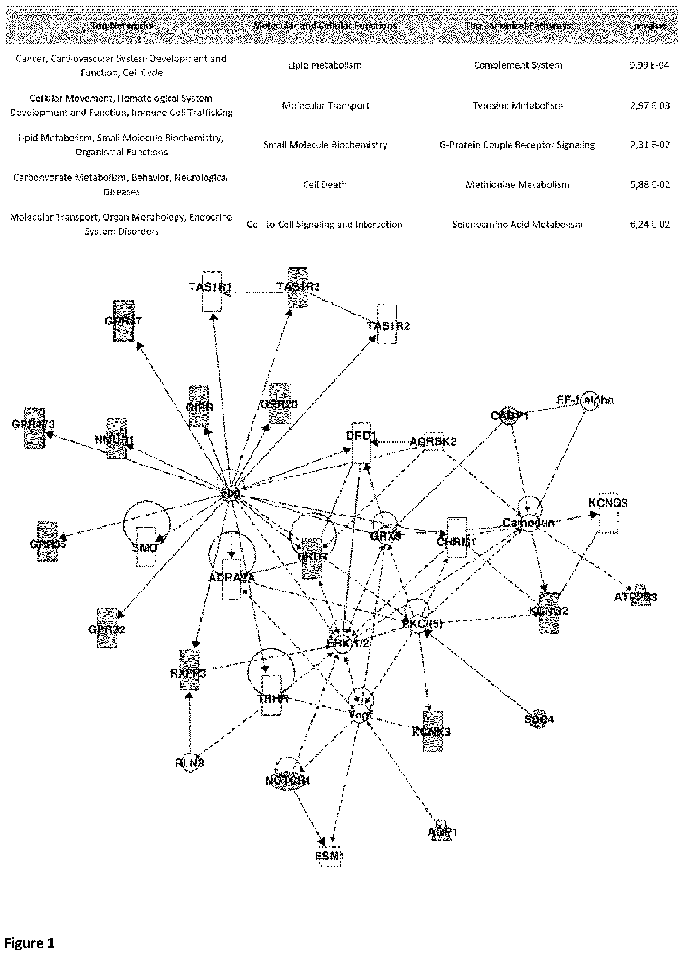

**Figure 1****.** Bioinformatic analysis using the Ingenuity Pathway Analysis (IPA) software for differential expression to interpret the biology of CTCs. Top image: Main networks, molecular and cellular functions together with the main canonical pathways and the corresponding p values. Bottom image: Interaction network for cell movement pathways, lipid metabolism and carbohydrates according to the expression profile of CTCs.

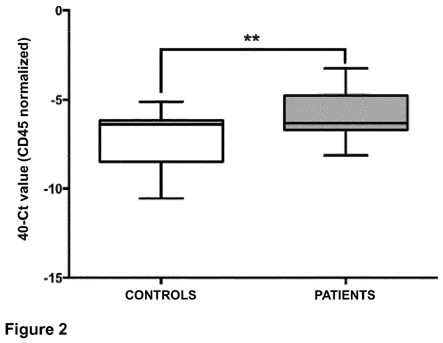

**Figure 2****.** Validation of TAS1R3 expression in circulating tumor cells isolated from patients with metastatic non-small-cell lung cancer (NSCLC) (n=42), compared to the control group of healthy donors (n=16).

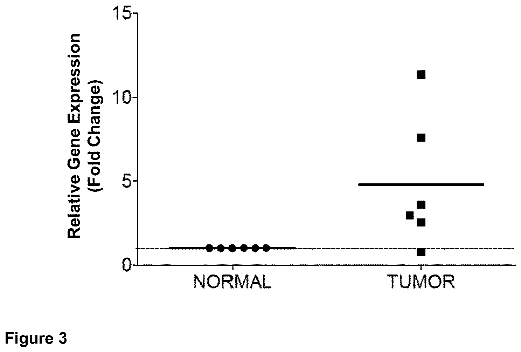

**Figure 3****.** TAS1R3 expression in tumor tissue from patients with metastatic lung cancer (NSCLC) versus non-tumor control tissue (n=6).

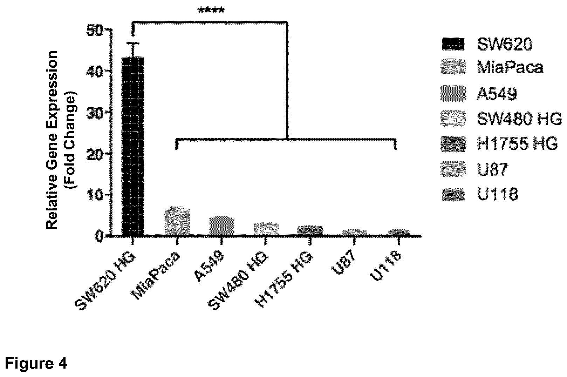

**Figure 4****.** Relative expression of TAS1R3 receptor (mRNA) in different cell lines of different tumor types, colon (SW620 and SW480), lung (A549 and H1755), glioblastoma (U87 and U118), and pancreas (MiaPaCa2), with reference to the values of the U87 line (P-value=0.0001).

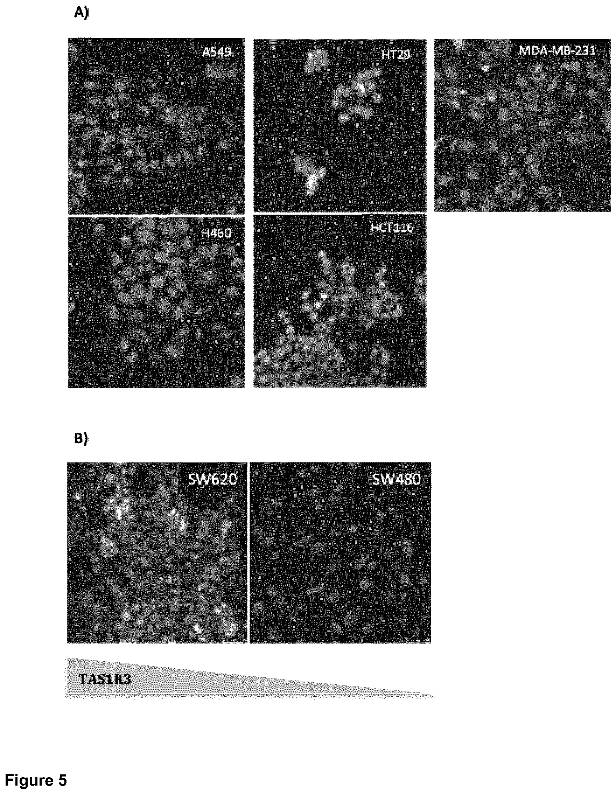

**Figure 5****.** Study of TAS1R3 expression by immunofluorescence in a panel of tumor cells of varying origin (lighter and dotted signal around the nucleus) (A). Immunofluorescence reveals a greater expression of the protein of interest, TAS1R3 (light signal), in colon cells of metastatic origin (SW620), versus colon cells isolated from primary tumor (SW480) (B). Cell nuclei are stained with DAPI.

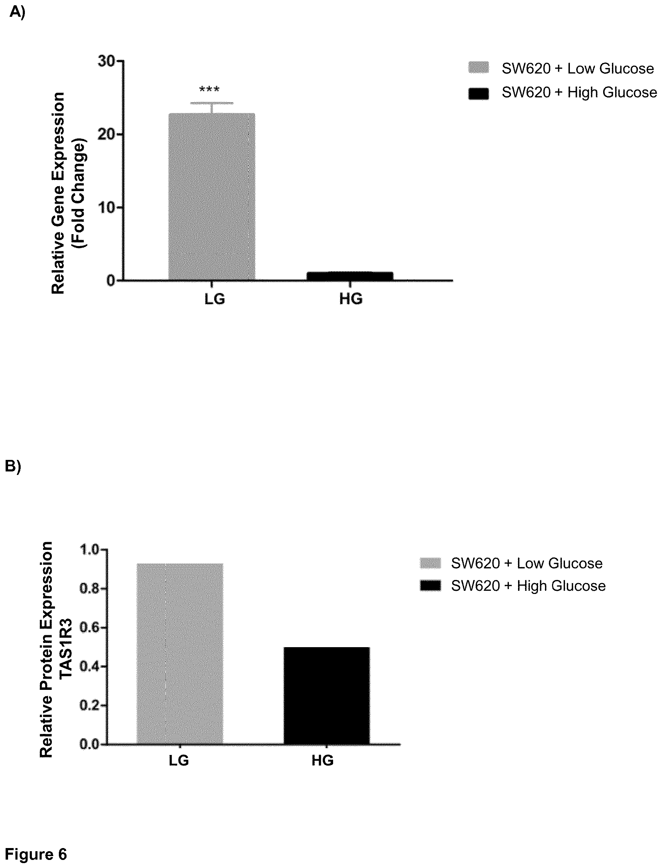

**Figure 6****.** TAS1R3 expression in SW620 cells cultured in medium with high and low glucose concentration. RT-PCR (A). Densitometric analysis of protein expression by Western Blot (B).

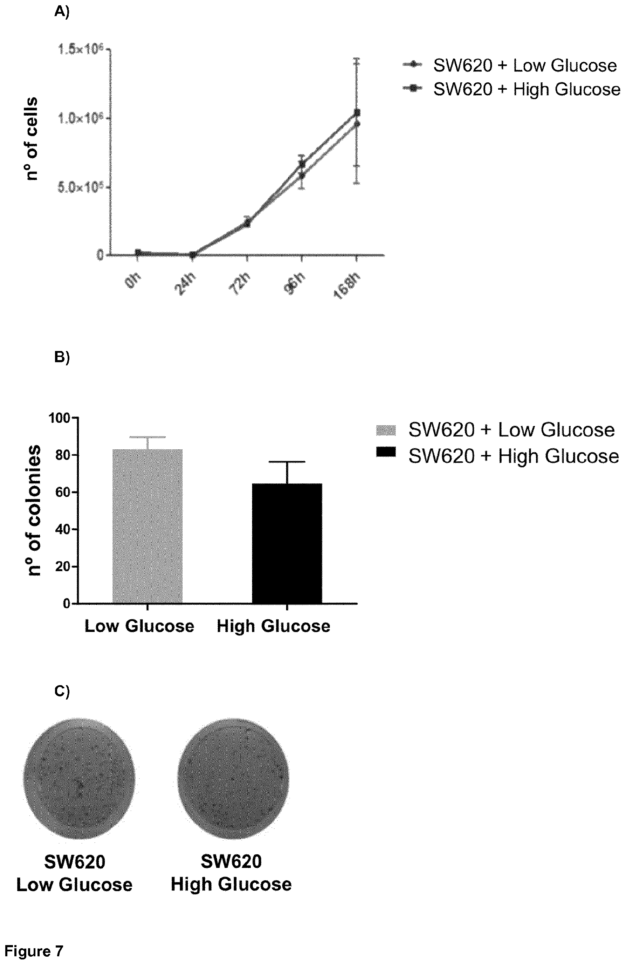

**Figure 7****.** Cell proliferation (A) and colony formation (B) assays of SW620 cell line cultured in medium with high and low glucose concentration (the data correspond to a number of 400 seeded cells that were kept in culture for 15 days).

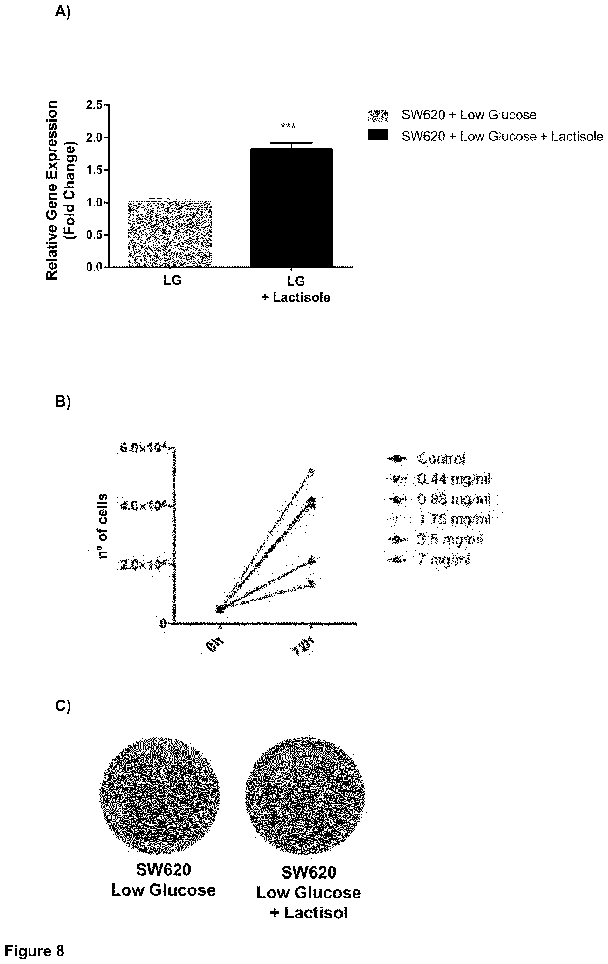

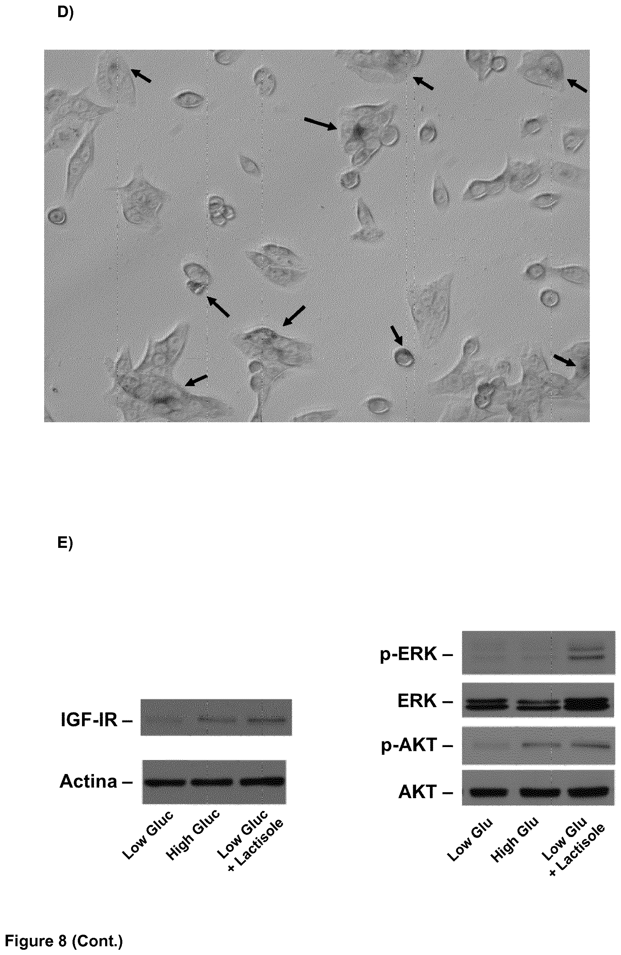

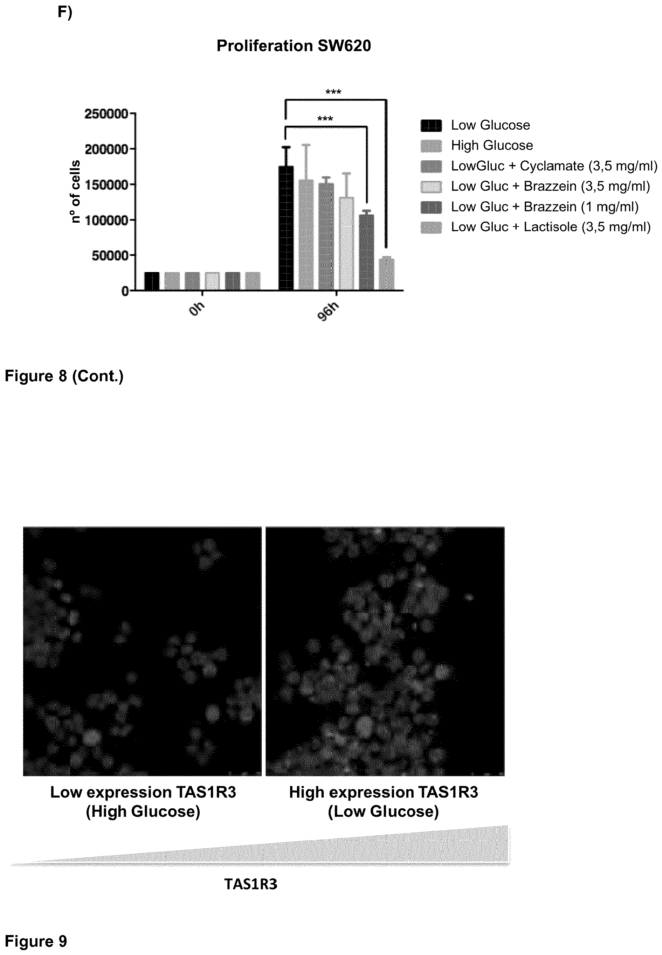

**Figure 8****.** RT-PCR of the receptor when the cells are kept in contact with lactisole for 9 days, comparing it with the controls (SW620 cultured in medium with low glucose concentration) (A). Cell proliferation of SW620 cells cultured in medium with low glucose concentration, and increasing concentrations of lactisole (from 0.44 mg/mL to 7 mg/mL) for 72h (B). Colony formation of control SW620 cells or in contact with the lactisole ligand, at a concentration of 3.5 mg/mL, after 15 days of culture (C). Beta-galactosidase staining, indicative of cellular senescence, in SW620 cells treated with lactisole (3.5 mg/mL for 72 hours, arrows show cells where staining can be seen) (D). Western blot showing the expression of different proteins involved in tumor progression in SW620 cells when they are exposed to different glucose concentrations (high and low) and lactisole (3.5 mg/ml) for 9 days (E). Cell proliferation of SW620 cells cultured in DMEM medium supplemented with different receptor ligands (glucose, cyclamate, brazzein and lactisole) (p-value<0.0001) (F).
**Figure 9****.** Internalization of sphingomyelin nanoemulsions functionalized with lactisole (O:SM:Lact 1:0.1:0.1), labeled with DiR and incubated for 4 hours in SW620 cells with higher expression of TAS1R3 (cultured in low glucose concentration) or with lower expression of TAS1R3 (cultured in high glucose concentration). The intensity of the fluorescence is greater in the case of functionalized nanoemulsions incubated with cells with higher expression of the receptor. Cell nuclei are stained with DAPI and the lightest signal is attributed to DiD, encapsulated within the nanoemulsions.

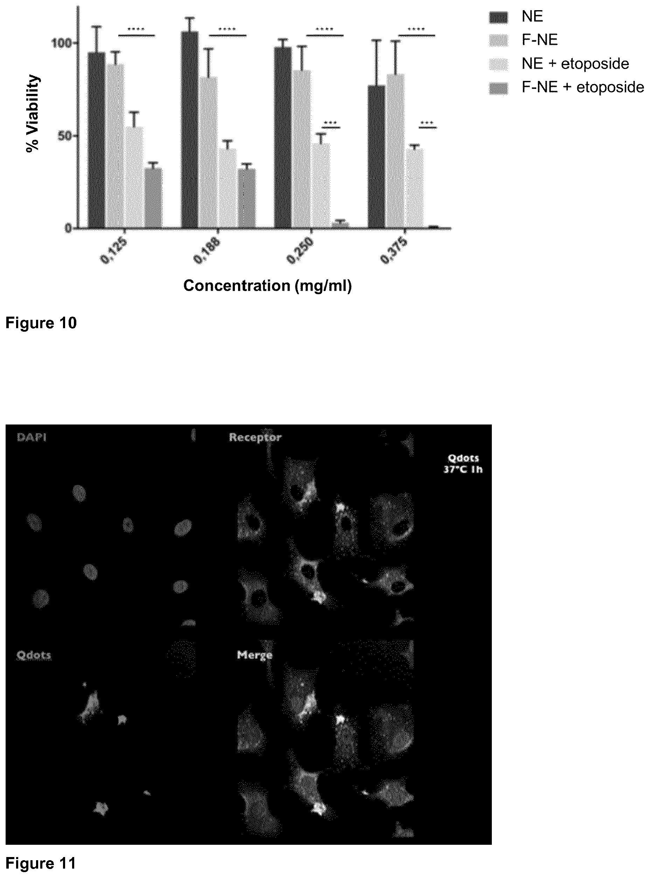

**Figure 10****.** Cell viability (MTT assay) of colon tumor cells (SW620) after being treated with white nanoemulsions of oleic acid and sphingomyelin (NE) and nanoemulsions of oleic acid and sphingomyelin functionalized with lactisole (F-NE), or encapsulating the antitumor drug etoposide, after 48 days of incubation (p-value, ****=0.0001, ***=0.001).
**Figure 11****.** Internalization of fluorescent quantum dots (red), functionalized with a human anti-TAS1R3 polyclonal antibody (Ref sc50353, SCBT). The quantum dots were incubated for 1 hour at 37 °C. The labeling of the TAS1R3 receptor (green) was performed in order to determine its co-localization with the quantum dots. Cell nuclei are stained with DAPI.

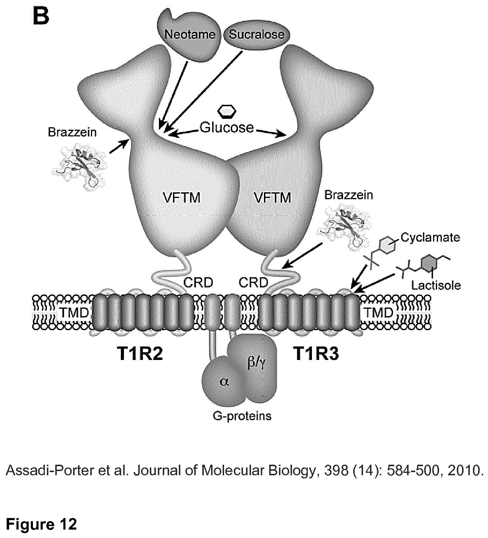

**Figure 12****.** Diagram of TAS1R3 receptor and its ligands.

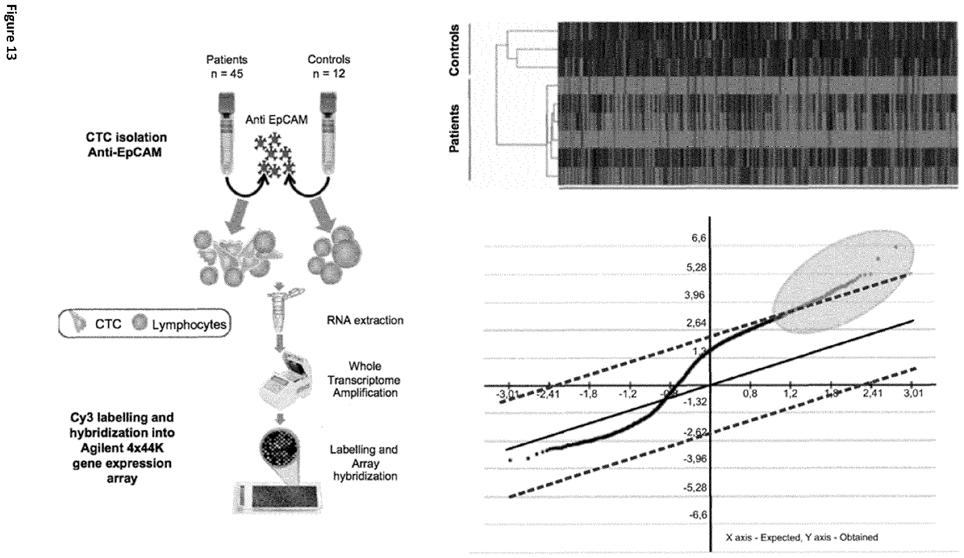

**Figure 13****. Detection of CTCs and gene expression profile.** Workflow diagram for the gene expression profile of CTCs isolated by immunotechniques from patients with advanced stage non-small-cell lung cancer. Hierarchical grouping of genes differentially expressed between patients (n=10) and controls (n=4) (specific genes of CTCs). Graph of the result of the analysis of significance for microarrays (Significance Analysis for Microarrays (SAM)), which shows the differences of gene expression between the group of patients and the controls. The highlighted points correspond to genes with increased expression levels and statistically significant in the group of patients compared to the control group, which is considered to characterize the population of metastatic lung cancer CTCs.

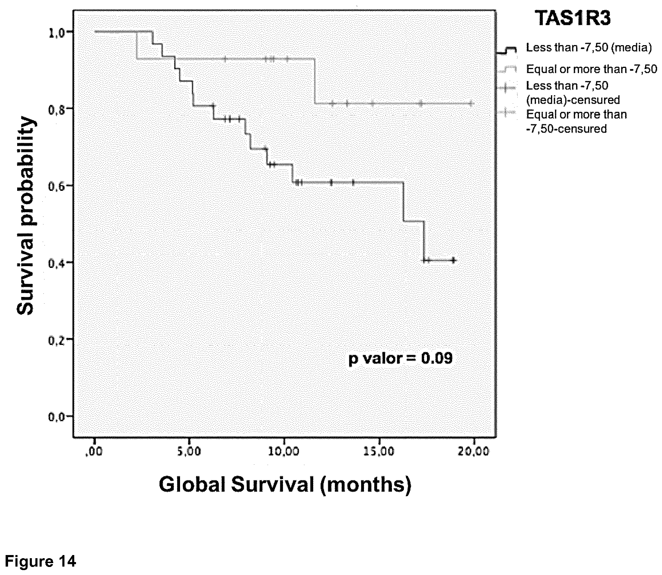

**Figure 14****. Kaplan-Meier curves.** They refer to overall survival based on the presence of high or low/moderate levels of TAS1R3 in the CTC samples of 42 patients with non-small-cell lung cancer. Patients' overall survival (OS) times were established as the time elapsed between the start of the chemotherapy line and the death of the patient. A greater survival is observed for patients with lower TAS1R3 receptor expression values (values≥-7.5, the cut-off point was set at the mean of the expression values of TAS1R3). The data analysis was carried out using the statistical analysis package SPSS.

### Detailed description of the invention

### Definitions

In the present invention, "TAS1R3 receptor" is understood as a taste G protein-coupled transmembrane receptor, which is expressed in taste buds, but also in other tissues such as liver and pancreas, and in tumor cells. There are multiple references to this receptor such as; HGNC: 15661 (NCBI Vega: OTTHUMG00000003071); Entrez Gene: 83756; Ensembl: ENSG00000169962; OMIM: 605865; o UniProtKB: Q7RTX0. It can be found forming heterodimers with the TAS1R1 receptor or TAS1R2 and act as a detector of sweet taste or umami, being activated by different amino acids, glucose, etc. See figure 12 for a diagram of the TAS1R3 receptor and its ligands.

In the present invention, a ligand for the TAS1R3 receptor is understood to be molecules capable of interacting with the TAS1R3 receptor, such as mono and disaccharides, artificial sweeteners such as sucralose, cyclamate, neoesperidine, dihydrochalcone, and derivatives, sweetness inhibitors such as lactisole and derivatives, proteins such as brazzein, or fragments thereof, as well as others specifically designed by selection systems such as antibodies, fragments of antibodies, peptides, aptamers, small molecules, proteins, etc.

In the present invention, "conjugates with ligands" are chemical conjugates that incorporate a ligand and a molecule with therapeutic activity (drug, radiopharmaceutical, etc.), or for diagnostic purposes (radioisotope, chelator, gadolinium, fluorophores, etc.).

In the present invention, "ligands functionalized with reactive groups" mean ligands that have been chemically modified to present a reactive group that can subsequently be used in a chemical or biochemical reaction, or for the subsequent binding or selective interaction of other molecules.

As used herein, the term "biological sample isolated from the patient" refers to a biological tumor sample or that comprises tumor tissue removed by a routine surgical procedure for diagnostic use in routine clinical protocol. Additionally, this term is understood to encompass any tissue or biological sample isolated from blood, serum or plasma comprising tumor tissue or circulating tumor cells or substances released by the tumor.

As used herein, the term "tissue involvement" refers to the presence of tumor cells in the tissue.

As used herein, the term "determination of TAS1R3 expression" refers to primers, antibodies or any other system that can evaluate the expression of said protein or any of its expression products such as RNA.

As used herein, the term "patient" refers, preferably, to a human. However, it can refer to a mammal, including non-primate mammals (e.g. horse, dog, cat, rat, mouse, cow or pig) and primate mammals (e.g., monkey).

As used herein, the term "molecular marker/tumor biomarker" refers to a biological marker of cancer, i.e., substance (s) produced by the tumor or by stromal cells closely related to the presence thereof, and that offers information of clinical interest on the state of tissue involvement affected by tumor cells. By "molecular marker/tumor biomarker" is also meant a biological marker of cancer, that is, substance (s) produced by the tumor or by stromal cells closely related to the presence thereof, and which offers the possibility of directing therapies against said cells through the use of receptor ligands, preferably ligands in the form of conjugates.

The determination of TAS1R3 expression levels can be carried out by immunological techniques such as for example, ELISA, ELONA, immunoblotting, immunofluorescence or immunohistochemistry, flow cytometry and aptahistochemistry. Immunoblotting is based on the detection of proteins previously separated by gel electrophoresis under denaturing conditions and immobilized on a membrane, generally nitrocellulose by incubation with a specific antibody and a development system (for example, chemiluminescence). Immunofluorescence analysis requires the use of an antibody specific for the target protein for the analysis of expression. ELISA and ELONA are based on the use of antigens, antibodies or aptamers labeled with enzymes so that conjugates formed between the target antigen and the labeled antibody result in the formation of enzymatically active complexes. Since one of the components (the antigen, or the labeled antibody or aptamer) is immobilized on a support, the antigen-antibody/aptamer complexes are immobilized on the support and thus, can be detected by the addition of a substrate that it is converted by the enzyme into a product that is detectable by, for example, spectrophotometry or fluorometry.

When an immunological method is used, any reagent, such as antibodies or aptamers, known to bind to target proteins with high affinity to detect the amount of target proteins, can be used. However, the use of an antibody is preferred, for example polyclonal sera, hybridoma supernatants or monoclonal antibodies, fragments of antibodies, Fv, Fab, Fab' and F(ab')2, scFv, diabodies, triabodies, tetrabodies and humanized antibodies. On the other hand, the determination of protein expression levels can be carried out by means of immunohistochemical techniques well known in the state of the art. To carry out the determination by immunohistochemistry, and/or suitable histochemistry, the sample can be a fresh, frozen or paraffin-embedded sample and fixed using a protective agent of the formalin type. For immunohistochemical determination, the sample is stained with an antibody or aptamer specific for TAS1R3 and the frequency of cells that have been stained and the intensity of staining determined. Typically, the sample is assigned a value indicative of the expression and total and that is calculated based on the frequency of stained cells (value that varies between 0 and 4) and of the intensity in each of the stained cells (variable value between 0 and 4). Typical criteria for assigning expression values to samples have been described in detail, for example, in Handbook of Immunohistochemistry and In Situ Hybridization in Human Carcinomas, M. Hayat E d., 2004, Academic Pres s. Additionally, immunohistochemistry makes it possible to identify which type of cells present in cancerous tissue are those that present altered levels of marker expression. Preferably, the immunohistochemical detection is carried out in parallel with cell samples that serve as a positive marker and as a negative marker and, as a reference, healthy tissues of the same origin as the tumor being analyzed can be used. It is also common to use a background control.

In those cases, in which a large number of samples is to be analyzed (for example, when several samples from the same patient or samples from different patients are to be analyzed), the use of matrix formats and/or automated procedures is possible. In one embodiment, the use of tissue microarrays (tissue microarrays or TMA) that can be obtained using different techniques is possible. The samples that are part of the microarrays can be analyzed in different ways including immunohistochemistry, in situ hybridization, in situ PCR, RNA or DNA analysis, morphological inspection and combinations of any of the above. Methods for processing tissue microarrays have been described, for example, in Konenen, J. et al., (Nat. Med. 1987, 4: 844-7). Tissue microarrays are prepared from cylindrical cores of 0.6 to 2 mm in diameter from tissue samples embedded in paraffin and re-embedded in a single receptor block. In this way, tissue from multiple samples can be inserted into a single block of paraffin. The determination of the expression levels of TAS1R3 needs to be correlated with the reference values corresponding to the median value of the TAS1R3 expression levels measured in a collection of tumor tissues in biopsy samples from subjects with cancer. Said reference sample is typically obtained by combining equal amounts of samples from a population of subjects. In general, typical reference samples will be obtained from subjects who are clinically well documented and in whom the disease is well characterized by one of the usual methods (digital rectal examination, occult blood test in the stool, sigmoidoscopy, colonoscopy, biopsy), determination of tumor markers such as carcinoembryonic antigen, ultrasound, CT, nuclear magnetic resonance, positron emission tomography). In such samples, the normal (of reference) concentrations of the biomarker can be determined, for example by providing the average concentration over the reference population. Several considerations are taken into account when determining the reference concentration of the marker. Such considerations include the type of sample involved (for example tissue or CSF), the age, weight, sex, general physical condition of the patient and the like. For example, equal amounts of a group of at least 2, at least 10, at least 100 to preferably more than 1000 subjects are taken as reference group, preferably classified according to the above considerations, for example of various age categories. The collection of samples from which the reference level derives will preferably be made up of subjects suffering from the same type of cancer as the patient under study.

Once this median or threshold value has been established, the level of this marker expressed in tumor tissues of patients with this median value can be compared, and in this way, be assigned to the "increased" or "decreased" level of expression. Due to the variability between subjects (for example, aspects related to age, race, etc.) it is very difficult (if not practically impossible) to establish absolute reference values of TAS1R3 expression. Thus, in a particular embodiment, the reference values for "increased" or "decreased" expression of TAS1R3 expression are determined by calculating the percentiles by conventional means involving testing the TAS1R3 expression levels in one or more isolated samples in subjects for whom the disease is well documented by one of the methods mentioned above. The "reduced" levels of APTX can then be assigned, preferably, to samples where the TAS1R3 expression levels are equal to or less than the 50th percentile in the normal population, including, for example, expression levels equal to or lower than the 60th percentile in the normal population, equal to or less than the 70th percentile in the normal population, equal to or less than the 80th percentile in the normal population, equal to or lower than the 90th percentile in the normal population, and equal to or lower than the 95th percentile in the normal population. The "increased" levels of TAS1R3 can then be preferably assigned to samples where the TAS1R3 expression levels are equal to or exceed the 50th percentile in the normal population, including, for example, expression levels equal to or in excess of the 60th percentile in the normal population, equal to or in excess of the 70th percentile in the normal population, equal to or in excess of the 80th percentile in the normal population, equal to or in excess of the 90th percentile in the normal population, and equal to or in excess of the 95th percentile in the normal population. The term "increased expression levels of TAS1R3", as used herein, refers to levels of TAS1 R3 higher than those appearing in a reference sample. In particular, a sample can be considered to have increased levels of TAS1R3 expression when the expression levels are, with respect to the reference sample, at least 1.1 times, 1.5 times, 5 times, 10 times, 20 times, 30 times, 40 times, 50 times, 60 times, 70 times, 80 times, 90 times, 100 times or even more with respect to the sample isolated from the patient.

### Description

The authors of the present invention have identified in CTCs (circulating tumor cells) (see example 1) and in tumors, a new receptor, TAS1R3 (a receptor of taste), and have determined that the expression of said receptor varies depending on the presence of glucose, and is related to the presence of primary, disseminated or metastatic tumor cells.

In this sense, as can be seen in Figures 2 to 5, a greater expression of TAS1R3 has been validated in circulating tumor cells isolated from patients with metastatic lung cancer (NSCLC) (n=42), compared to the control group of healthy donors (n=16) (figure 2), a higher expression of TAS1R3 in tumor tissue of patients with metastatic lung cancer (NSCLC), versus non-tumor control tissue (n=6) (figure 3), and expression of the TAS1R3 receptor in different cell lines of different tumor types, colon (SW620 and SW480), lung (A549 and H1755), glioblastoma (U87 and U118), and pancreas (MiaPaCa2) (figure 4). Likewise, the expression of TAS1R3 has been studied by immunofluorescence in a panel of tumor cells of different origin, in particular in colon cells of metastatic origin (SW620), compared to primary tumor isolated colon cells(SW480).

These data demonstrate the diagnostic potential of this biomarker, given that Figures 2 and 3 illustrate a clear differentiation in the levels of expression in tumor cells versus non-tumor control tissue, and its prognostic potential, based on the results of Figure 14, that show a better overall survival in those patients with lower levels of TAS1 R3 receptor expression.

The present invention, therefore, relates to a new prognostic/diagnostic tumor biomarker capable of detecting the presence of primary, disseminated or metastatic tumor cells in a biological sample such as a tissue or a sample of blood, serum or plasma. Also, the invention relates to the detection of TAS1 R3 expression levels in a biological sample such as a tissue or a serum or plasma blood sample, to diagnose the involvement or presence in said tissue or biological sample of tumor cells, whether primary, disseminated or metastatic. Said information would also have a prognostic value since the identification of metastatic tumor cells or the comparison of the level and/or concentration of the TAS1R3 receptor in a biological sample such as a tissue or a sample of blood serum or plasma with the level and/or concentration of the TAS1 R3 receptor in a biological sample from a healthy individual, or from a part of a healthy organ or tissue or biological sample without tumor involvement, or from a reference value, would provide useful information in an individual's prognosis such as information about the overall survival (OS) of a patient suffering from cancer. In this sense, the determination of the expression of TAS1R3 and the identification of the cell type that expresses it, will allow predicting the involvement of the tissue affected by tumor cells. Additionally, the expression levels of the TAS1R3 receptor will allow predicting the possible involvement implied by metastatic tumor cells, thus providing a clear prognostic value.

Therefore, a first aspect of the present invention refers to a method for the prediction or *in vitro* diagnosis of tumor involvement or presence (neoplastic or metastatic involvement or presence) in a patient, characterized by comprising the following steps:
a) starting from at least one biological sample isolated from the patient, preferably a sample of blood, serum or plasma or a sample of tissue, preferably fixed, and more preferably, a sample fixed and embedded in paraffin; and
b) determining the level of expression of TAS1R3 in the cells of a biological sample such as a tissue or a sample of blood serum or plasma, by means of a cell type identification technique; and
c) comparing said level of expression with the level of expression of the TAS1R3 receptor in a biological sample of a healthy individual, or of a part of a healthy organ or tissue or biological sample without tumor involvement, or with a reference value,
wherein an increased level of expression compared to the level of expression of the TAS1R3 receptor in a biological sample of a healthy individual, or of a part of a healthy organ or tissue or biological sample without tumor involvement, or with a reference value, is indicative of the tumor involvement or presence (neoplastic or metastatic involvement or presence) in a patient.

Additionally, in a second aspect, the present invention refers to a method for the *in vitro* prognosis of the clinical course of a patient suffering from a tumor involvement or presence (neoplastic or metastatic involvement or presence), characterized by comprising the following steps:
a) starting from at least one biological sample isolated from the patient, preferably a sample of blood, serum or plasma or a sample of tumor tissue, preferably fixed, and more preferably, a fixed sample and included in paraffin; and
b) determining the expression of TAS1 R3 in the cells of a biological sample such as a tissue or a serum or plasma blood sample, by means of a cell type identification technique, and
c) comparing said level of expression with the level of expression of the TAS1R3 receptor in a biological sample from the same patient obtained at an earlier moment in time than the biological sample of section a), or with a reference value,
wherein an increased level of expression compared to the level of expression of the TAS1R3 receptor in a biological sample from the same patient obtained at an earlier moment in time than the biological sample of part a), or with a reference value, is indicative of a negative clinical evolution of the patient.

Negative clinical evolution of the patient is understood as, preferably, an estimated overall survival of less than 5 years, preferably less than 3 years.

Additionally, a third aspect the present invention refers to a method for the *in vitro* monitoring of the clinical evolution of a patient suffering from a tumor involvement or presence (neoplastic or metastatic involvement or presence), characterized by comprising the following steps:
a) starting from at least one biological sample isolated from the patient, preferably a sample of blood, serum or plasma or a sample of tumor tissue, preferably fixed, and more preferably, a fixed sample and included in paraffin; and
b) determining the expression of TAS1 R3 in the cells of a biological sample such as a tissue or a sample of blood, serum or plasma, by a cell type identification technique, and
c) comparing said level of expression with the level of expression of the TAS1R3 receptor in a biological sample from the same patient obtained at an earlier moment in time than the biological sample of section a), or with a reference value,
wherein an increased level of expression compared to the level of expression of the TAS1R3 receptor in a biological sample from the same patient obtained at an earlier moment in time than the biological sample of part a), or with a reference value, is indicative of a negative clinical evolution of the patient.

Additionally, a fourth aspect the present invention refers to a method for monitoring the response to *in vitro* treatment of a patient suffering from a tumor involvement or presence (neoplastic or metastatic involvement or presence), characterized by comprising the following steps:
a) starting from at least one biological sample isolated from the patient, preferably a sample of blood, serum or plasma or a sample of tumor tissue, preferably fixed, and more preferably, a sample fixed and embedded in paraffin; and
b) determining the expression of TAS1 R3 in the cells of a biological sample such as a tissue or a serum or plasma blood sample, by means of a cell type identification technique, and
c) comparing said level of expression with the level of expression of the TAS1R3 receptor in a biological sample of the same patient obtained at an earlier moment in time than the biological sample of section a), or with a reference value,
wherein an increased level of expression compared to the level of expression of the TAS1R3 receptor in a biological sample from the same patient obtained at an earlier moment in time than the biological sample of part a), or with a reference value, is indicative of a lack of the patient's response to treatment.

As already stated in the "definitions" section, as used herein, the term "determination of the expression of TAS1R3" refers to antibodies or any other system that can evaluate the expression of said protein, such as aptamers. In order to obtain antibodies or aptamers useful in the determination of TAS1 R3 expression levels, a sequence analysis of the human TAS1R3 protein was carried out. From these data, it was possible to predict that the sequences most suitable from the immunological point of view (present in the N-terminal, extracellular region) would be the following:
Acetyl-LSQQLRMKGDYVLGGC-amide (SEQ ID NO 1)
Acetyl-ERLKIRWHTSDNQKPVSRC-amide (SEQ ID NO 2)

Based on these data, the peptide was synthesized (Acetyl-LSQQLRMKGDYVLGGC-Amide) using the heterologous expression system Escherichia coli. The resulting protein was purified to homogeneity with a final purity of 90-95%, 2 mg conjugated to KLH, 3 mg conjugated to BSA. The design of the genomic construct was carried out based on the literature (Maitrepierre et al., 2012). Likewise, a study of storage buffer formulation was carried out to preserve the integrity of the protein (restricting the use of detergents and polysaccharides in the final formula). The batch obtained was aliquoted in labeled sterile vials and stored at -80°C until use. After several immunizations of the mice with 4 plus 2 doses of 50 µg of peptide, the bleeds were titrated in order to evaluate the increase of the response against the peptide. The results show that the mice generated some response against the peptide, although with very low values that discourage the achievement of production.

Therefore, in a preferred embodiment of the first to fourth aspect of the invention, the determination of the expression of TAS1 R3 in the cells of a biological sample such as a tissue or a sample of blood serum or plasma, is carried out with antibodies or fragments thereof or aptamers capable of binding to SEQ ID NO 1 or SEQ ID NO 2.

In a preferred embodiment of the first to fourth aspect of the invention, the present invention is characterized in that the negative predictive value of said method is greater than 80%, more preferably greater than 90%, 95%, 96%, 97%, 98% or 99%. That is, when the expression of TAS1 R3 is not detected in the cells in the biological sample of the patient, it means that said sample will not be affected by the tumor.

As used herein, the term "negative predictive value" refers to the percentage of cases that having the negative test results in not having the disease (tissue involvement affected by tumor cells).

In another preferred embodiment, the present invention is characterized in that the patient suffers from a solid tumor, preferably suffers from a disease selected from among the following group: breast cancer, melanoma, uveal melanoma, pancreatic cancer, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma.

In another preferred embodiment, the present invention is characterized in that the cell type identification technique defined in step b) is selected from among the following group: immunohistochemistry, immunofluorescence, aptahistochemistry, and flow cytometry. Preferably, the technique for cell type identification defined in step b) is immunohistochemistry, and more preferably, immunohistochemistry comprises the following steps:
i) pretreatment of the sample,
ii) immunostaining;
iii) counterstaining; and
iv) immunostaining analysis.

In another preferred embodiment, the present invention is characterized in that the sample already obtained from a tissue defined in step a) is selected from among the following group: fixed tissue sample, fresh tissue sample, and frozen tissue or blood sample, serum or plasma. Preferably, it is a fixed tissue sample or a sample of blood, serum or plasma.

In another preferred embodiment, the present invention is characterized in that the patient is a mammal. Preferably, the mammal is selected from the following group: human, primate and non-primate. More preferably, the patient is a human.

The present invention also relates, in a fifth aspect of the invention, to TAS1 R3 for use as a tumor biomarker, preferably in the method described above.

The present invention also relates to a kit for use in the method described above.

The embodiment of the invention can be carried out in fresh tissue, fixed or frozen by flow cytometry, immunohistochemistry, immunofluorescence or any other technique that allows the identification of the cell type known by a person skilled in the art.

On the other hand, and due to the enormous potential of this receptor for the selective direction of molecules, such as nanoparticles, drugs, or radioisotopes, and taking into account that we know of the existence of molecules that interact with said receptor, and are not endogenous, and, therefore, competition phenomena do not occur; we have selected several ligands of the receptor, in particular several sweeteners, to validate the hypothesis that it is possible to design therapies against it. We have also functionalized nanostructures with said ligands, to direct them against tumors that express TAS1 R3, such as primary tumors or disseminated tumor cells such as CTCs (circulating tumor cells) and metastatic cells. We have also observed that some ligands, such as lactisole, or nanostructures functionalized with it, can give rise to therapeutic effects, in terms of tumor toxicity and proliferation.

In order to carry out said functionalization, the ligands described in Table 2 were acquired, and either they were joined by covalent binding to a hydrophobic moiety (e.g. LACT-C16), or by incubation on preformed nanoemulsions (ex. BRA). The sequences of the peptides used and the physicochemical properties of the resulting nanoemulsions are described below (Table 2).

**Table 1. Composition and physicochemical properties of functionalized nanoemulsions with several ligands of interest.**

| **LIGAND** | **DESCRIPTION** | **Size* (nm)** | **PDI*** | **Superficial Charge* ζ (mV)** |
|---|---|---|---|---|
| LACT | Lactisole (N° CAS 150436-68-3)-C16 / Lactisole-C18 | 139±8 | 0.2 | -59±4 |
| BRA | CFYDEKR | 161±4 | 0.2 | -33±2 |

| | | | | |
|---|---|---|---|---|
| LACT: Lactisole; BRA: brazzein peptide; PDI: Polidispersity Index ζ: Zeta Potential *NANOPARTICLES. | | | | |

Once the previous functionalization was carried out, we observed a high internalization capacity of those functionalized nanoemulsions with ligands against TAS1R3 in cells that express the receptor (tumor cells). For example, in the case of nanoemulsions functionalized with lactisole, the DiD signal encapsulated in the nanoemulsions was greater than with respect to those other non-functionalized nanoemulsions. In addition, we observed that when tumor cells expressed the target receptor with less intensity (in this case TAS1R3 in SW620 cells cultured in medium with higher glucose content (low expression, example 2)), the intensity of the signal decreased (example 4).

The present invention demonstrates, therefore, that the functionalization of nanosystems with ligands against TAS1R3, expressed in the cell membrane of tumor cells, in particular in the membrane of disseminated and metastatic tumor cells, makes it a unit with a strong therapeutic potential. In particular, said nanosystems can be used as vehicles for the development of combination therapies. Additionally, said potential to functionalize structures other than nanoemulsions, such as quantum dots, are described in Example 5 of the present invention. This discovery can be extrapolated to any type of nanosystem, such as nanoparticles, micelles, or liposomes, i.e. ligands against this receptor can serve as selective vehicles that serve to functionalize any type of nanosystem. Furthermore, this discovery can be extrapolated to any type of system, such as any ligand-drug conjugate and/or ligand-radioisotope comprising ligands against this receptor and can serve as selective intracellular vehicles that functionalize said drug or radioisotope.

**Table 2. Composition and physicochemical properties of functionalized nanostructures.**

| **LIGAND** | **DESCRIPTION** | **COMPOSITION** | **Size (nm)** | **PDI** | **Superficial Charge ζ (mV)** |
|---|---|---|---|---|---|
| BRA | CFYDEKR | O:SM:PC:BRA 1:0,1:0,1:0,005 | 161±4 | 0.2 | -33±2 |
| LACT | Lactisole (N° CAS 150436-68-3)-C16 / Lactisole-C18 | O:SM:LACT 1:0,1:0,1 | 139±8 | 0.2 | -59±4 |
| | | O:SM:LACT1:0,1:0,1 | 139±8 | 0.2 | -59±4 |
| | | LACT | 149±22 | 0.5 | -53±10 |
| | | O:LACT 1:01 | 175±5 | 0.2 | -71±5 |
| | | O:LACT 1:0,2 | 155±15 | 0.2 | -65±4 |
| | | V: LACT 1:0,1 | 148±4 | 0.3 | -71±5 |

| | | | | | |
|---|---|---|---|---|---|
| SM: Sphingomyelin; O: oleic acid; PC: phosphatidylcholine; LACT: Lactisole-C16/-C18; BRA: brazzein peptide. PDI: Polidispersity Index. ζ: Zeta Potential. | | | | | |

Therefore, the present invention demonstrates that once any type of nanostructure, liposome, drug or radioisotope with ligands has been functionalized against the receptor of the present invention, a vehiculation of said structure is observed towards those cells that express the receptor, in particular a vehiculation towards primary, disseminated or metastatic tumor cells.

Thus, a sixth aspect of the invention relates to the use of the TAS1 R3 receptor as a molecular marker/tumor biomarker to direct therapies against tumor cells through the use of receptor ligands, preferably ligands in the form of conjugates or immunotoxins. The immunotoxins are conjugates of a specific target agent, typically an antibody or fragment thereof directed to the tumor, with a cytotoxic agent, such as a toxin moiety. The target agent is designed to direct the toxin to the cells that carry the target antigen, and kill such cells.

The present invention, therefore, in a seventh aspect of the invention, provides conjugates of:
- a specific target agentcapable of interacting with the TAS1 R3 receptor, such as mono and disaccharides, artificial sweeteners such as sucralose, cyclamate, neoesperidine, dihydrochalcone, and derivatives, sweetness inhibitors such as lactisole, and derivatives, proteins such as brazzein, as well as others specifically designed by means of selection systems such as antibodies, fragments of antibodies, peptides, aptamers, small molecules, proteins,
- with a cytotoxic agent, such as a cytotoxic agent or an antitumor drug, a radioisotope, a nanostructure or a nanoemulsion.

In a preferred embodiment of the seventh aspect of the invention, the target agent are antibodies or fragments thereof, or peptides, or aptamers capable of binding to SEQ ID NO 1 or SEQ ID NO 2.

An eighth aspect of the invention relates to the conjugate of the seventh aspect of the invention, for use in therapy or *in vivo* diagnosis. Preferably, for use in the *in vivo* treatment or diagnosis of cancer, more preferably, for use in the treatment or *in vivo* diagnosis of a solid tumor, preferably a tumor selected from among the following group: breast cancer, melanoma, uveal melanoma, pancreatic cancer, lung cancer, prostate cancer, stomach cancer, head and neck cancer, sarcoma, glioblastoma, neuroblastoma, colon and rectal cancer, head and neck cancer, kidney and bladder cancer, and hepatocarcinoma, or disseminated or metastatic cells.

Throughout the description and claims the word "comprises" and its variants do not intend to exclude other technical characteristics, additives, components or steps. For those skilled in the art, other objects, advantages and characteristics of the invention will emerge partly from the description and partly from the practical implementation of the invention. The following figures and examples are provided by way of illustration, and are not intended to be limiting of the present invention.

### Examples

### Example 1. Expression of TAS1R3 receptor in tumor cells.

The analysis of the gene expression of the TAS1R3 receptor in CTCs (circulating tumor cells) of patients with metastatic lung cancer was performed from peripheral blood samples (patients=10, controls n=4), using magnetic particles for their isolation (EpCAM-based CELLectionTM Epithelial Enrich Dynabeads^{®} kit), following the diagram shown in Figure 13. After extracting the RNA from the samples, they were hybridized in gene expression arrays. The signal was captured and processed in order to obtain a list of differentially expressed genes in patients with respect to the controls, among which were the TAS1R3 receptor. In summary, the total RNA of CTCs was amplified, the complementary DNA was hybridized in gene expression microarrays (Agilent) and the raw data were processed giving rise to 2,392 points, (7.01%) that met the criteria of quality. The average signal was 60,889 units, with 76,597 and 16,979 units for the patient and control groups, respectively. After normalization, 1,810 probes were considered with expression greater than 211 in at least 8 patients and 2 controls for subsequent analyzes. The genes that characterize the population of CTCs isolated from patients with advanced non-small-cell lung cancer were identified by applying the software MeV v4.7 (Multiexperiment Viewer) (TM4 Microarray Software Suite). Two classes of microarray data analysis (SAM) algorithms were used for the selection of candidate genes with strict criteria to obtain significance. Assuming that the Delta parameter for the median of False Discovery Rate (FDR)=0 is 3.3, a list of 2461 genes was presented, considering only the 529 probes with a log2 ratio greater than 1.5, which characterizes specifically the population of CTCs of patients with advanced NSCLC. The hierarchical grouping (HCL) was performed with these genes expressed significantly using MeV. The Pearson correlation was chosen as a distance metric, using the absolute distance and the complete link grouping parameters in the MeV options.

A genomic analysis of differential expression was also carried out. As depicted in Figure 1, the gene sets were first analyzed with the Ingenuity Pathway (IPA) software to generate gene networks and provide an overview of the signaling pathways that characterize the CTCs of patients with metastatic lung cancer. Of the 529 significant genes, 349 were annotated genes and, therefore, identified by IPA. Consistently with an actively disseminated subpopulation of tumor cells, the main molecular and cellular functions represented by the set of specific genes of CTCs were related to cell movement, cell death, signaling and cell-to-cell interaction, molecular transport and lipid metabolism, mainly orchestrated by the signaling of the receptor coupled to G proteins and the pathways of the complement system. The list of genes was further filtered in terms of 1) the function related to adhesion, cell movement and migration and then 2) the location on the cell surface. The resulting molecules were Nocth1 and TAS1R3.

Next, we proceeded to validate the expression of the candidate genes in an independent cohort of patients and controls. Using quantitative PCR, it was confirmed that the expression levels of this receptor were significantly higher in the patients (Figure 2). The expression values were normalized with CD45 (hematopoietic cell marker) as non-specific isolation marker. In addition, a complementary validation of the expression of this receptor was performed in samples of paraffinized healthy and tumor tissue (Figure 3). To do this, we performed RNA extraction from 5 paraffin sections of 14 microns each using a specific extraction kit for paraffinized samples (RNeasy FFPE kit, QIAGEN). In order to know the concentration of it, the Nanodrop equipment (Nanodrop 2000C, Thermoscientific) was used. With these data, the normalization of the RNA concentration in each sample was carried out, placing a total of 2 µg, in a final volume of 10 µL with nuclease-free water. Next, the High Capacity cDNA Reverse Transcription Kit (Appliesbiosystems) was used and a thermal cycler (peq STAR 96HPL, VWR^{®}) was used for the passage of RNA to cDNA. Once the cDNA was obtained, the mixture was carried out with the probe specific for TAS1R3 together with the Taqman Universal PCR MasterMix. GAPDH was used as control or housekeeping, and the polymerase chain reaction was carried out in StepOne Plus-Real-Time PCR system, AppliedBiosystems^{®}.

To obtain information about the prognostic value of the marker, a Kaplan-Meier survival analysis was performed using the SPSS statistical package (Figure 14). To apply this method, all survival times observed are ordered from lowest to highest, noting for each of them the number of deaths and censures produced. For each period of time the probability of survival is calculated, and the Kaplan-Meier function is "the probability of individual survival accumulated over time". The overall survival (measured in months) of patients with CTC with TAS1R3 expression values lower than the cut -7.5 is better (clear line) compared to patients with CTCs with higher expression values of TASR13 (dark line), p-value of 0.09.

Regarding the evaluation of expression in cell cultures, several types of cell lines were used, obtained from ATCC (American Type Culture Collection) (Figure 4). Metastatic colon cells from the lymph node (SW620, CCL-227), colorectal adenocarcinoma epithelial cells (SW480, CCL-228), lung carcinoma epithelial cells (A549, CCL-185), and metastatic lung cells from the liver (H1755, CRL-5892), pancreatic carcinoma cells (MIA PaCa-2, CRL-1420), and glioblastoma cells (U118, HTB-15 and U87MG, HTB-14) were used. The SW620, SW480, A549, U118 and U87MG lines were maintained in DMEM HG medium (Dulbecco'sModifiedEagle'sMedium - High glucose, Sigma-Aldrich), and the H1755 line in RPMI 1640 medium (Gibco^{®}, LifeTecnologies), both supplemented to the 10% with fetal bovine serum (Gibco^{®}, LifeTecnologies) and 1% with antibiotic (penicillin and streptomycin, Sigma-Aldrich). For the quantitative analysis of the expression of TAS1R3, a polymerase chain reaction was carried out with reverse transcriptase (RT-PCR), starting from RNA extracted from all cell lines in culture. First, the cells were routinely trypsinized, and then counting them using the Neubauer chamber and thus being able to isolate 5 million cells from each line to start from a comparable cell number. RNA extraction was carried out using the extraction kit (GeneJET RNA Purification Kit, ThermoScientic), and the analysis was carried out in a manner similar to that described in a previous paragraph. Once the procedure was finished, the data were analyzed. The results of expression are shown in Figure 4 and 5. The metastatic cancer cell lines isolated from the lymph node (SW620) show a greater expression thereof, in relation to the isolated line of primary tumor of the same patient, SW480. These differences are not observed when we refer to lung adenocarcinoma cells, where both lines, regardless of their origin (metastatic lung H1755 cells in liver and A549 cells from lung carcinoma) present similar expression levels. The pancreatic adenocarcinoma cell line, MiaPaCa-2, has slightly higher levels of expression than the rest (except in relation to SW620). While the glioma lines (U87MG and U118) barely express the receptor to a lesser degree.

Regarding the immunofluorescence studies shown in figure 5, the cells under study were seeded one day before the test was carried out in 8-well micro-chambers (PLC30108, SPL LifeSciences). After 24 hours, the culture medium was removed by aspiration and washed with PBS 1X, to proceed to fix the cells with para-formaldehyde 4%, for 15 minutes at room temperature. They were washed with PBS 1X twice, and then the cells were permeabilized with 0.2% triton 100X, for 10 minutes at room temperature. After washing the triton, the primary antibody anti-human (rabbit) (MBiosource) was incubated in 0.2% BSA, dissolved in 1X PBS in the 1:1000 dilution, incubating the plate for one hour at room temperature. The primary antibody was then washed, and the anti-rabbit secondary antibody (ab150077 or 111-585-144 Jackson Immunoresearch) was applied, dilution 1:500 in BSA 0.2% PBS1X, which is labeled with the fluorophore (Alexa fluor 488 or 594 depending on the antibody). At the same time, Hoechst 33342 (ThermoFisher^{®}) (dilution 1:1000) was added and incubated for one hour, protecting the fluorophore from possible light degradation. Once the incubation time had passed, it was again washed with PBS1X three times under stirring, to then remove the walls of the micro-chamber, apply the Mowiol mounting medium (Calbiochem) and place the coverslip on the sample. It was left overnight to dry at room temperature protected from darkness, and the next day it was stored at -20 °C until its observation in the confocal microscope (Laser Microscope Confocal Leica SP8^{®}). As seen in Figure 5A, most cell lines of tumor origin have detectable levels of fluorescence. When comparing the intensity between the colon lines SW480 (primary) and SW620 (ganglion metastasis) (5B), it is possible to observe a greater intensity in the SW620 cell line, so the results obtained in this test are correlated with the data of RT-PCR.

For the qualitative study of proteins, Western Blot was performed from proteins isolated from the SW620 cell line, and the results are shown in Figure 6 (B). Cells were cultured in medium with high (Dulbecco's Modified Eagle's Medium-High glucose, Sigma-Aldrich) and low (Dulbecco's Modified Eagle's Medium -Low glucose, Sigma-Aldrich) glucose concentration, passing them with a normal frequency. The cells were trypsinized and a count was performed using the Neubauer chamber, to have a similar number of cells (5 million) of all the lines. It was then centrifuged at 150 xg for 5 minutes at room temperature (Centrifuge SL 16R, rotor TX-400, ThermoScientific^{®}), aspirating the medium and re-suspending the pellet obtained in PBS 1X to subsequently perform a new centrifugation under the same conditions that the first, aspiring the PBS 1X and trying to leave the pellet as isolated as possible. This pellet was then re-suspended in complete 1X RIPA (8 µL Sodium fluoride (NaF), 1.6 µl sodium orthovanadate (Na₃OV₄), 2 µL protease inhibitors (PIC), 2 µL phenylmethylsulfonyl fluoride (PMSF) and 186,4µl RIPA 1X), incubating on ice for 20 minutes, followed by sonication for 10 minutes, after which it was centrifuged at 13800 xg, 15 minutes at 4 °C (Microcentrifuge 5415R, rotor F452411 Eppendorf^{®}), collecting the supernatant from the samples. The total protein of each sample was quantified by the Bradford method (conversion method of bicathionic to monocationic copper in alkaline conditions influenced by specific amino acids such as cysteine, cystine, tryptophan, tyrosine and also by the peptide chain; the amount of bicathionic copper will depend of the amount of these components and will be determined spectrophotopically) using the commercial kit DC-Assay (BIO-RAD^{®}) and establishing a calibration line with bovine serum albumin (BSA, Sigma-Aldrich). The absorbance of the samples was determined on a VersaMax ELISA Microplate Reader (Molecular Devices^{®}) plate reader at 750 nanometers. Vertical gels of 1.5 mm thickness were prepared for electrophoresis, consisting of a concentrating gel, 4% SDS-PAGE (sodium dodecyl sulfate-polyacrylamide), and a 10% separating gel of SDS-PAGE. At the same time, the sample was prepared with a total amount of 20 µg of protein, 6X loading buffer (Tris pH 6.8, SDS, glycerol, bromophenol blue and beta-mercaptoethanol) and a volume of water depending on the protein concentration of each sample. After denaturing the sample at 95 °C for 5 minutes in a thermoblock, the sample was loaded into the wells of the gels placed in the Mini-Protean II (Bio-Rad) cuvette, and a current of 50 V was applied until the sample reached the limit of the gel separator, to then increase this voltage to 120 V, an estimated time of 1 h and 30 min (until the front leaves the gel). At all times, the gel was embedded in running buffer. The proteins were then transferred to a 0.45 µm nitrocellulose membrane (BioRad), contacting the electrophoresis gel with this membrane, and applying a voltage of 100V for 1h and 30min, in transfer buffer. After the process, the membrane was bathed with Ponceau Red solution, to observe the presence of the proteins. It was subsequently blocked with milk in TTBS 1X (tris buffer saline + Tween20) at 5%, for one hour under stirring and at room temperature. After this time, the membranes were incubated with the primary antibody specific for that protein (at the concentrations and dilutions of the Santa Cruz commercial house), after introduction of the membrane in a special plastic bag, and it was kept overnight under stirring at 4 °C. The next day, the membrane was removed and 3 washes were made for 10 minutes under stirring with 1X TTBS, and then the membrane was returned to another plastic bag for incubation with the secondary antibody (anti-mouse or anti-rabbit, BD -Jackson) diluted 1:5000 in TTBS1 X, keeping the membrane under stirring for one hour at room temperature. At the end of the incubation with the secondary antibody, 3 washes of 10 minutes were again performed under stirring with 1X TTBS at room temperature. Finally Pierce ECL reagent (Enhanced chemioluminiscence) Western Blotting Substrate (Thermo Fisher) 1:1 was applied on the membrane, and its development proceeded. The membrane was introduced with the ECL in the revelation cassette, in the dark, and the autoradiographic film (Mamorray) was placed on it, at various exposure times, using the Curix 60 automatic developer, AGFA^{®}. The data obtained using the imaged software was relativized, after scanning the films.

### Example 2. Expression of TAS1R3 in culture media with different levels of glucose concentration.

The expression of TAS1R3 in the colon line SW620 varies depending on the glucose content in the culture medium (high levels of glucose give rise to a lower expression, in relation to that observed in Figure 6A for cells cultured in low content of glucose). This result, additionally, we have confirmed by western blot (Figure 6B). Moreover, as seen in Figure 6, receptor expression increases when SW620 colon cells are grown in the absence of glucose, which relates the expression of this receptor to cellular metabolism.

Cell proliferation studies were carried out in SW620 cells, Figure 7A, cultured in medium with high and low glucose concentration. The cells were trypsinized and plated the same number of cells in order to count them at times 0, 24, 72, 96 and 168 hours, using for this the Neubauer chamber, after trypsinization thereof. In an analogous manner, cell proliferation studies were carried out in medium with the presence of free ligand, figure 8, which was added to the culture medium at different concentrations. Photos were taken using the microscope (Leica DMIL^{®} microscope) and subsequently they were trypsinized, and the number of cells was counted to make the comparison between these and the starting ones in order to observe the effect of the ligand on the cells. Cell proliferation was comparable under the study conditions. However, when studies of colony formation were carried out, starting from an amount of 200 cells per well and waiting for 15 days, it was observed that cells cultured in medium with low concentration of glucose, and that express higher levels of receptor, formed more colonies than those grown in high glucose medium (Figure 7B). For colony formation studies, 12 well plates were used, plating in different amounts of cells (200, 400,600 and 800 cells per well), in medium with high (4500 mg/L) and low (1000 mg/L) content of glucose. These plates were observed by optical microscopy and with the naked eye, for 15 days, until the size of the colonies was visible without the use of a microscope. A solution of MTT dissolved in PBS (5 mg/mL) was then added to the wells themselves, and after 4 hours of incubation, the medium was cautiously aspirated, where the purple-stained colonies would remain on the bottom of the well. The plates were allowed to dry overnight at room temperature, and then scanned, in order to count the colonies in each of the wells using the OpenCFU program.

### Example 3. TAS1R3 as a target for the development of new anti-tumor therapies.

The presence of lactisole in the culture medium, a ligand of the TAS1 R3 receptor, causes an increase in the expression of the receptor under study (Figure 8A). Although at higher concentrations, the presence of ligand interferes with cell proliferation (Figure 8B), a fact that we have related to the induction of cellular senescence (Figure 8D). In addition, we have also seen by western blot that the presence of ligands of TAS1R3 such as lactisole or glucose (HG) in the culture medium of SW620, activates pathways related to tumor progression, such as pERK, p-AKT or IGF-IR (Figure 8E). It was also determined how other ligands for TAS1 R3, a peptide derived from brazzein and cyclamate, also interfere with proliferation, to a greater or lesser extent depending on the concentration and each molecule (Figure 8F).

To determine whether after cell incubation with the ligand cell senescence is induced, 500,000 cells of the SW620 line were cultured in 6-well plates and medium with low glucose concentration. In several wells, ligand (3.5 mg/mL) was added. After 72 hours, the wells were washed and fixed (2% formaldehyde, 0.2% glutaraldehyde in PBS) for 15 minutes at room temperature. The solution was eliminated giving three washes with 1X PBS, and then the staining solution was applied, calculated for one milliliter of solution: 200 µL citric acid/phosphate buffer, pH 6.0 (dibasic sodium phosphate (Na₂HPO₄) 0.2 M + 0.1 M citric acid ), 30 µL 5 M NaCl, 50 µL potassium ferricyanide (K₃[Fe(CN)₆]) 100 mM, 50 µL potassium ferrocyanide (K₄[Fe(CN)₆]) 100 mM, 2 µL magnesium chloride (MgCl₂) 1 M, 50 µL solution X-gal (20 mg/mL of 5-bromo-4-chloro-3-indolyl β D-galactopyranoside in dimethylformaldehyde) and 618 µL distilled water. It was incubated in the oven at 37°C overnight, the wells were washed with distilled water in motion twice, to then apply PBS 1X and observe the possible senescent cells under the Vert microscope. A1, Zeiss^{®}. Blue staining is observed, corresponding to cells in senescence.

For the test of colony formation in media with the free ligand, the same process was carried out but in this case, the cells were seeded in 1000 ml/L of glucose (low glucose concentration) and 3.5 mg/mL of ligand was added to each of the 24 wells. The results are shown in Figure 8C. I n the case of the treated cells, no representative colony formed on the plate was observed, as opposed to control SW620 cells that grew in medium with low glucose concentration (LG). Therefore, it can be said that the ligand affects tumor proliferation and consequently the ability to form colonies.

This finding is extremely interesting, since it could present this ligand with a dual function, for the active direction of nanoparticles and as a drug.

### Example 4. Targeted therapies to TAS1R3.

The functionalization of the nanoemulsions with different ligands against TAS1R3 took place as described below. Nanoemulsions were obtained spontaneously after adding 100µL of ethanol, containing oleic acid and sphingomyelin (5mg and 500µg respectively), together with 500µg of lactisole covalently linked to a C16-C18 chain, on 1 mL of Milli-Q water (MilliporeMilli-Q system^{®}) under gentle magnetic stirring. High-performance liquid chromatography (HPLC) was used to confirm the inclusion of the ligand in the nanoemulsions using an HPLC system (1260 Infinity II, Agilent) equipped with a G7111A pump, a G7129A autosampler and a G7114A UV-Vis detector, with an InfinityLab Poroshell 120EC-C18 column (Agilent, 4.6× 00 mm, 4 µm pore size).

To study the effect of functionalization on the ability of nanoemulsions to interact with cells that express the receptor of interest, flow cytometry and confocal microscopy techniques were used. Specifically, SW620 cells were used, and confocal microscopy was used. For these experiments, 80,000 cells were seeded per well in 8-well micro-chambers (PLC30108, SPL LifeSciences). After 24 hours, the cells were incubated with nanoemulsions prepared from oleic acid and a sphingomyelin derivative labeled with nitrobenzoxadiazole (NBD), which in turn encapsulated DiR (OLM; O:SM1:0.1), and those same nanoemulsions functionalized with lactisole (OLM-L; O:SM:Lact 1:0.1:0.1), at a final concentration in the nanoemulsion well of 0.12 mg/mL. After 4 hours of incubation at 37 °C, the cells were washed with 1X PBS twice, and then fixed with 4% paraformaldehyde for 15 minutes, after which the wells were washed twice with PBS 1X, and then Cell nuclei were stained with Hoechst 33342 (Thermo Fisher^{®}). The mounting medium (Mowiol, Calbiochem) was applied and the samples were observed under the confocal microscope (Laser Microscope Confocal Leica SP8^{®}). A similar experiment was carried out comparing in this case the internalization of the functionalized nanoemulsions with lactisole (O:SM:Lact 1:0.1:0.1), and labeled with DiD, after incubation in SW620 cells with cultured medium with high or low concentration of glucose, and therefore with different levels of expression of the receptor, as seen in example 2. It was found that indeed the intensity of fluorescence due to nanoemulsions decreased in the case of incubation on SW620 cells with lower expression of TAS1R3 receptor for lactisole, as can be seen in Figure 9.

O:SM:Lact 1:0.1:0.1 (NE-F), loaded with etoposide at 1% by weight, with respect to the other components. 10,000 cells were seeded per well in a 96-well plate. After 24 h of culture, 20 µL of the test formulation was applied to 110 µL of culture medium, in order of increasing concentration, establishing as controls a positive one, adding the vehicle in which the nanosystem is dissolved (water in the largest part of the cases), and a negative one, or total death, where a dilution of Triton 100X at 6% was applied. After 48 hours, the culture medium of the plate was aspirated, washing with 1X PBS, then applying the MTT reagent at a concentration of 5 mg/mL in 1X PBS, after dilution 1:10 in DMEM medium without supplementation , and filtered with a 0.22 µm filter. 110 µL was applied per well. After 4 hours in the incubator, the medium was removed from the plate, and a volume of 110 µL of 1X DMSO (dimethylsulfoxid, 99.7%, AcrosOrganics) was added to dissolve the formazan crystals originated by the mitochondrial enzymes. Protecting the light plate was incubated 15 minutes at 37 °C, to then measure the absorbance at 570 nanometers in the spectrophotometer (Multiskan EX, ThermoLabsystems^{®}) and obtain the EC50 values of each formulation, using the GraphPad Prism 5 program. For the encapsulation of an antitumor drug, etoposide was selected, and 13.75µl of a solution of 40 mg/mL (550 µg of drug) was added to the organic phase. All nanoemulsions were isolated by centrifugation for 30 minutes at 14000 xg 15 °C (Microcentrifuge 5415R, rotor F452411 Eppendorf^{®}), in order to eliminate all that was not part of the nanoemulsions. As shown in Figure 10 A, greater cytotoxic activity was observed in the case of cells treated with the drug nanoemulsions (the white ones barely showed activity), in particular in the case of functionalized nanoemulsions.

### Example 5. Experiment with Quantum dots

Quantum dots functionalized with an antibody against the human protein TAS1R3. The antibody against TAS1R3 (Ref sc50353, SCBT) was conjugated to quantum dots using the SiteClick^{™} Qdot^{®} 655 Antibody Labeling Kit (Ref S10453, ThermoFisher) following the supplier's instructions. The concentration in the final preparation was determined by measuring the optical density at the specified wavelength and then using the formula A = εcL, where A is the absorbance, ε is the molar extinction coefficient, c is the molar concentration, and L is the length of the path. We checked the size distribution of the quantum dots, observing the presence of a majority population of approx. 400 nm.

We performed an interaction test of functionalized quantum dots with lung tumor cells A549 in suspension and adherence. Incubation was carried out at 4 °C and 37 °C for 4 hours. After the incubation time, the culture medium was removed and washed with 1X PBS. The cells incubated in suspension were seeded a posteriori to allow their adherence and perform immunofluorescence studies of the TAS1R3 receptor. After 24 hours, the culture medium was removed by aspiration and washed with PBS 1X, to proceed to fix the cells with 4% paraformaldehyde, for 15 minutes at room temperature. Then, the incubation of the anti-human primary antibody TAS1R3 (rabbit) (Ref sc50353, SCBT), in 0.2% BSA, dissolved in 1X PBS in the 1:1000 dilution, incubating the plate for one hour at room temperature was carried out. The primary antibody was then washed, and the anti-rabbit secondary antibody (111-545-144 Jackson Immunoresearch) was applied, dilution 1:500 in BSA 0.2% PBS1X, which is labeled with the fluorophore (Alexa Fluor 488). At the same time, Hoechst 33342 (ThermoFisher^{®}) (dilution 1:1000) was added and incubated for one hour, protecting the fluorophore from possible light degradation. Once the incubation time had passed, it was washed again with PBS1X three times under stirring, to then remove the walls of the micro-chamber, apply the Mowiol mounting medium (Calbiochem) and place the coverslip on the sample. It was left overnight to dry at room temperature protected from the dark, and the next day it was observed under a confocal microscope (Leica SP8^{®} Confocal Laser Microscope.

## Claims

1. *In vitro* use of the TAS1R3 membrane cell receptor as a tumor biomarker.

2. Method for the *in vitro* diagnosis of tumor presence, neoplasia or metastatic presence in a patient, which comprises the following steps:
a) starting from at least one biological sample isolated from the patient, determining the level of expression of TAS1R3 in the cells of said biological sample, and
c) comparing said level of expression with the level of expression of the TAS1R3 receptor in a biological sample of a healthy individual, or of a part of a healthy organ or tissue or biological sample without tumor presence, or with a reference value,
wherein an increased level of expression compared to the level of expression of the TAS1R3 receptor in a biological sample of a healthy individual, or of a part of a healthy organ or tissue or biological sample without tumor presence, or with a reference value, is indicative of tumor presence, neoplasia or metastatic presence in a patient.

3. Method for the *in vitro* prognosis of the clinical course of a patient who suffers from tumor presence, neoplasia or metastatic presence in a patient, which comprises the following steps:
a) starting from at least one biological sample isolated from the patient, determining the expression of TAS1R3 in the cells of said biological sample, and
c) comparing said level of expression with the level of expression of the TAS1R3 receptor in a biological sample from the same patient obtained at a time earlier from the time in which the biological sample of part a) was obtained, or with a reference value,
wherein an increased level of expression compared to the level of expression of the TAS1R3 receptor in the biological sample obtained at a time earlier from the time in which the biological sample of part a) was obtained, or with a reference value, is indicative of a negative clinical evolution of the patient.

4. Method for the *in vitro* monitoring of the clinical course of a patient suffering from tumor presence, neoplastic or metastatic presence, which comprises the following steps:
a) starting from at least one biological sample isolated from the patient, determining the expression of TAS1R3 in the cells of said biological sample, and
c) comparing said level of expression with the level of expression of the TAS1R3 receptor in a biological sample from the same patient obtained at a time earlier from the time in which the biological sample of part a) was obtained, or with a reference value,
wherein an increased level of expression compared to the level of expression of the TAS1R3 receptor in a biological sample from the same patient obtained at a time earlier from the time in which the biological sample of part a) was obtained, or with a reference value, is indicative of a negative clinical evolution of the patient.

5. Method for monitoring the response to treatment of a patient suffering from tumor presence, neoplastic or metastatic presence, which comprises the following steps:
a) starting from at least one biological sample isolated from the patient, determining the expression of TAS1R3 in the cells of said biological sample, and
c) comparing said level of expression with the level of expression of the TAS1R3 receptor in a biological sample from the same patient obtained at a time earlier from the time in which the biological sample of part a) was obtained, or with a reference value,
wherein an increased level of expression compared to the level of expression of the TAS1 R3 receptor in a biological sample from the same patient obtained at a time earlier from the time in which the biological sample of part a) was obtained, or with a reference value, is indicative of lack of response to treatment.

6. The method according to any of claims 2 to 5, wherein the determination of the expression of TAS1R3 in the cells of a biological sample is carried-out with aptamers, antibodies or fragments thereof from the list consisting of Fv, Fab, Fab 'and F(ab')2, scFv, or with diabodies, triabodies, tetrabodies and/or humanized antibodies capable of selectively binding to the TAS1R3 receptor.

7. The method according to claim 6, wherein said aptamers, antibodies or fragments thereof, or diabodies, triabodies, tetrabodies and/or humanized antibodies, are capable of selectively binding to any of the sequences SEQ ID NO 1 or SEQ ID NO 2.

8. The method according to any of claims 2 to 7, wherein the tumor present is a solid tumor, or wherein the patient has a disease selected from the group consisting of: breast cancer, melanoma, uveal melanoma, pancreatic cancer, lung cancer, prostate cancer, stomach cancer, head and neck cancer, sarcoma, glioblastoma, neuroblastoma, cancer of the colon and rectum, cancer of the head and neck, renal and bladder cancer, and hepatocarcinoma.

9. Use of the cellular membrane receptor TAS1R3 as a molecular marker/tumor biomarker to direct therapies against tumor cells through the use of ligands of the receptor, preferably ligands in the form of conjugates or immunotoxins.

10. The cellular membrane receptor TAS1R3 for use as a molecular marker/tumor biomarker for the *in vivo* diagnosis, through the use of ligands of the receptor, preferably ligands in the form of conjugates with radiopharmaceuticals; wherein ligand of the TAS1 R3 receptor are those molecules capable of binding the TAS1R3 receptor selected from mono and disaccharides; artificial sweeteners such as sucralose; cyclamate; neoesperidin; dihydrochalcone; sweetness inhibitors such as lactisole; brazzein; antibodies or fragments of antibodies.

11. A conjugate that comprises:
a) a specific target agent capable of binding the TAS1R3 receptor,and,
b) a cytotoxic agent, a radioisotope, a nanostructure or a nanoemulsion;
wherein the specific target agent capable of binding the TAS1R3 receptor is selected from the list consisting of: mono and disaccharides; artificial sweeteners such as sucralose; cyclamate; neoesperidin; dihydrochalcone; sweetness inhibitors such as lactisole; brazzein; antibodies or fragments of antibodies; for use in therapy against tumor cells or for use as a tumor biomarker *in vivo* diagnosis.

12. The conjugate for use according to claim 10, wherein the target agent is selected from the list consisting of: aptamers, antibodies, fragments of antibodies consisting of: Fv, Fab, Fab' and F(ab')2, scFv, or diabodies, triabodies, tetrabodies and/or humanized antibodies, wherein said target agents are capable of selectively binding to any of the sequences SEQ ID NO 1 or SEQ ID NO 2.

13. The conjugate for use according to any of claims 11 to 12, wherein said use is for the treatment or *in vivo* diagnosis of a solid tumor, preferably a tumor selected from the following group: breast cancer, melanoma, uveal melanoma, cancer pancreas, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma.

## Patentansprüche

1. *In*-*vitro*-Verwendung des TAS1R3-Membranzellrezeptors als Tumorbiomarker.

2. Verfahren für die *In-vitro*-Diagnose von Anwesenheit von Tumoren, Neoplasie oder Anwesenheit von Metastasen in einem Patienten, welches die folgenden Schritte umfasst:
a) ausgehend von mindestens einer biologischen Probe, welche aus dem Patienten isoliert wurde, das Bestimmen des Expressionsniveaus von TAS1R3 in den Zellen der genannten biologischen Probe, und
c) das Vergleichen des genannten Expressionsniveaus mit dem Expressionsniveau des TAS1R3-Rezeptors in einer biologischen Probe eines gesunden Individuums, oder eines Teils eines gesunden Organs oder Gewebes oder einer biologischen Probe ohne Anwesenheit von Tumoren, oder mit einem Referenzwert,
wobei ein erhöhtes Expressionsniveau im Vergleich zum Expressionsniveau des TAS1R3-Rezeptors in einer biologischen Probe eines gesunden Individuums, oder eines Teils eines gesunden Organs oder Gewebes oder einer biologischen Probe ohne Anwesenheit von Tumoren, oder mit einem Referenzwert, auf die Anwesenheit von Tumoren, Neoplasie oder Anwesenheit von Metastasen in einem Patienten schließen lässt.

3. Verfahren für die *In*-*vitro*-Prognose des klinischen Verlaufs eines Patienten, welcher unter Anwesenheit von Tumoren, Neoplasie oder Anwesenheit von Metastasen in einem Patienten leidet, welches die folgenden Schritte umfasst:
a) ausgehend von mindestens einer biologischen Probe, welche aus dem Patienten isoliert wurde, das Bestimmen der Expression von TAS1R3 in den Zellen der genannten biologischen Probe, und
c) das Vergleichen des genannten Expressionsniveaus mit dem Expressionsniveau des TAS1R3-Rezeptors in einer biologischen Probe aus dem gleichen Patienten, welche zu einem früheren Zeitpunkt erhalten wurde als der Zeitpunkt, zu welchem die biologische Probe des Teils a) erhalten wurde, oder mit einem Referenzwert,
wobei ein erhöhtes Expressionsniveau im Vergleich zum Expressionsniveau des TAS1R3-Rezeptors in der biologischen Probe, welche zu einem früheren Zeitpunkt erhalten wurde als der Zeitpunkt, zu welchem die biologische Probe des Teils a) erhalten wurde, oder mit einem Referenzwert, auf eine negative klinische Entwicklung des Patienten schließen lässt.

4. Verfahren für die *In*-*vitro*-Überwachung des klinischen Verlaufs eines Patienten, welcher unter der Anwesenheit von Tumoren, Anwesenheit von Neoplasie oder Metastasen leidet, welches die folgenden Schritte umfasst:
a) ausgehend von mindestens einer biologischen Probe, welche aus dem Patienten isoliert wurde, das Bestimmen der Expression von TAS1R3 in den Zellen der genannten biologischen Probe, und
c) das Vergleichen des genannten Expressionsniveaus mit dem Expressionsniveau des TAS1R3-Rezeptors in einer biologischen Probe aus dem gleichen Patienten, welche zu einem früheren Zeitpunkt erhalten wurde als der Zeitpunkt, zu welchem die biologische Probe des Teils a) erhalten wurde, oder mit einem Referenzwert,
wobei ein erhöhtes Expressionsniveau im Vergleich zum Expressionsniveau des TAS1R3-Rezeptors in einer biologischen Probe aus dem gleichen Patienten, welche zu einem früheren Zeitpunkt erhalten wurde als der Zeitpunkt, zu welchem die biologische Probe des Teils a) erhalten wurde, oder mit einem Referenzwert, auf eine negative klinische Entwicklung des Patienten schließen lässt.

5. Verfahren für die Überwachung der Behandlungsreaktion eines Patienten, welcher unter Anwesenheit von Tumoren, Anwesenheit von Neoplasie oder Metastasen leidet, welches die folgenden Schritte umfasst:
a) ausgehend von mindestens einer biologischen Probe, welche aus dem Patienten isoliert wurde, das Bestimmen der Expression von TAS1R3 in den Zellen der genannten biologischen Probe, und
c) das Vergleichen des genannten Expressionsniveaus mit dem Expressionsniveau des TAS1R3-Rezeptors in einer biologischen Probe aus dem gleichen Patienten, welche zu einem früheren Zeitpunkt erhalten wurde als der Zeitpunkt, zu welchem die biologische Probe des Teils a) erhalten wurde, oder mit einem Referenzwert,
wobei ein erhöhtes Expressionsniveau im Vergleich zum Expressionsniveau des TAS1R3-Rezeptors in einer biologischen Probe aus dem gleichen Patienten, welche zu einem früheren Zeitpunkt erhalten wurde als der Zeitpunkt, zu welchem die biologische Probe des Teils a) erhalten wurde, oder mit einem Referenzwert, auf einen Mangel von Behandlungsreaktion schließen lässt.

6. Verfahren nach einem der Ansprüche 2 bis 5, wobei die Bestimmung der Expression von TAS1R3 in den Zellen einer biologischen Probe mit Aptameren, Antikörpern oder Fragmenten derselben aus der Liste bestehend aus Fv, Fab, Fab' und F(ab')2, scFv, oder mit Diakörpern, Triakörpern, Tetrakörpern und/oder humanisierten Antikörpern, welche in der Lage sind, sich an den TAS1R3-Rezeptor selektiv zu binden, durchgeführt wird.

7. Verfahren nach Anspruch 6, wobei die genannten Aptamere, Antikörper oder Fragmente derselben, oder Diakörper, Triakörper, Tetrakörper und/oder humanisierten Antikörper, in der Lage sind, sich an eine der Sequenzen SEQ ID NO 1 oder SEQ ID NO 2 selektiv zu binden.

8. Verfahren nach einem der Ansprüche 2 bis 7, wobei der vorhanden Tumor ein solider Tumor ist, oder wobei der Patient eine Krankheit hat, ausgewählt aus der Gruppe bestehend aus: Brustkrebs, Melanom, Uvealmelanom, Bauchspeicheldrüsenkrebs, Lungenkrebs, Prostatakrebs, Magenkrebs, Kopf-Hals-Krebs, Sarkom, Glioblastom, Neuroblastom, Dickdarm- und Mastdarmkrebs, Kopf-Hals-Krebs, Nieren- und Blasenkrebs und Hepatokarzinom.

9. Verwendung des Zellmembranrezeptors TAS1R3 für dessen Verwendung als molekularer Marker/Tumorbiomarker, um Therapien gegen Tumorzellen zu richten, durch die Verwendung von Liganden des Rezeptors, vorzugsweise Liganden in Form von Konjugaten oder Immunotoxinen.

10. Zellmembranrezeptor TAS1R3 für dessen Verwendung als molekularer Marker/Tumorbiomarker für die *In-*vivo-Diagnose, durch die Verwendung von Liganden des Rezeptors, vorzugsweise Liganden in Form von Konjugaten mit Radiopharmazeutika;
wobei die Liganden des TAS1R3-Rezeptors solche Moleküle sind, welche in der Lage sind, sich an den TAS1R3-Rezeptor zu binden, ausgewählt aus Mono- und Disacchariden; synthetischen Süßstoffen wie Sucralose; Cyclamat; Neohesperidin; Dihydrochalcon; Süßehemmstoffen wie Lactisol; Brazzein; Antikörpern oder Fragmenten von Antikörpern.

11. Konjugat, welches Folgendes umfasst:
a) ein spezifisches Zielagens, welches in der Lage ist, sich an den TAS1R3-Rezeptor zu binden, und,
b) ein zytotoxisches Mittel, ein Radioisotop, eine Nanostruktur oder eine Nanoemulsion,
wobei das spezifische Zielagens, welches in der Lage ist, sich an den TAS1R3-Rezeptor zu binden, aus der Liste bestehend aus: Mono- und Disacchariden; synthetischen Süßstoffen wie Sucralose; Cyclamat; Neohesperidin; Dihydrochalcon; Süßehemmstoffen wie Lactisol; Brazzein; Antikörpern oder Fragmenten von Antikörpern ausgewählt wird;
für dessen Verwendung bei der Therapie gegen Tumorzellen oder für dessen Verwendung als Tumorbiomarker bei der *In-vivo*-Diagnose.

12. Konjugat für dessen Verwendung nach Anspruch 10, wobei das Zielagens aus der Liste bestehend aus: Aptameren, Antikörpern, Fragmenten von Antikörpern bestehend aus: Fv, Fab, Fab' und F(ab')2, scFv, oder Diakörpern, Triakörpern, Tetrakörpern und/oder humanisierten Antikörpern ausgewählt wird, wobei die genannten Zielagenzien in der Lage sind, sich an eine der Sequenzen SEQ ID NO 1 oder SEQ ID NO 2 selektiv zu binden.

13. Konjugat für dessen Verwendung nach einem der Ansprüche 11 bis 12, wobei die genannte Verwendung für die Behandlung oder *In*-*vivo*-Diagnose eines soliden Tumors, vorzugsweise eines Tumors ausgewählt aus der folgenden Gruppe: Brustkrebs, Melanom, Uvealmelanom, Bauchspeicheldrüsenkrebs, Lungenkrebs, Prostatakrebs, Magenkrebs, Kopf-Hals-Krebs, Sarkom, Glioblastom, Neuroblastom, Dickdarm- und Mastdarmkrebs, Kopf-Hals-Krebs, Nieren- und Blasenkrebs und Hepatokarzinom ist.

## Revendications

1. Utilisation *in vitro* du récepteur cellulaire de membrane TAS1R3 en tant que biomarqueur de tumeur.

2. Procédé pour le diagnostic *in vitro* de la présence de tumeur, de néoplasie ou de présence métastatique chez un patient, qui comprend les étapes suivantes :
a) à partir d'au moins un échantillon biologique isolé du patient, déterminer le niveau d'expression de TAS1R3 dans les cellules dudit échantillon biologique, et
c) comparer ledit niveau d'expression avec le niveau d'expression du récepteur TAS1R3 dans un échantillon biologique d'un sujet sain, ou d'une partie d'un organe ou d'un tissu ou d'un échantillon biologique sain sans présence de tumeur, ou avec une valeur de référence,
dans lequel un niveau d'expression accru par rapport au niveau d'expression du récepteur TAS1R3 dans un échantillon biologique d'un sujet sain, ou d'une partie d'un organe ou d'un tissu ou d'un échantillon biologique sain sans présence de tumeur, ou avec une valeur de référence, est révélateur de présence de tumeur, de néoplasie ou de présence métastatique chez un patient.

3. Procédé pour le pronostic *in vitro* de l'évolution clinique d'un patient qui souffre de la présence de tumeur, de néoplasie ou de présence métastatique chez un patient, qui comprend les étapes suivantes :
a) à partir d'au moins un échantillon biologique isolé du patient, déterminer l'expression de TAS1R3 dans les cellules dudit échantillon biologique, et
c) comparer ledit niveau d'expression avec le niveau d'expression du récepteur TAS1R3 dans un échantillon biologique du même patient obtenu à un moment antérieur au moment où l'échantillon biologique de la partie a) a été obtenu, ou avec une valeur de référence,
dans lequel un niveau d'expression accru par rapport au niveau d'expression du récepteur TAS1R3 dans l'échantillon biologique obtenu à un moment antérieur au moment où l'échantillon biologique de la partie a) a été obtenu, ou avec une valeur de référence, est révélateur d'une évolution clinique négative du patient.

4. Procédé pour le suivi *in vitro* de l'évolution clinique d'un patient souffrant de présence de tumeur, présence néoplasique ou métastatique, qui comprend les étapes suivantes :
a) à partir d'au moins un échantillon biologique isolé du patient, déterminer l'expression de TAS1R3 dans les cellules dudit échantillon biologique, et
c) comparer ledit niveau d'expression avec le niveau d'expression du récepteur TAS1R3 dans un échantillon biologique du même patient obtenu à un moment antérieur au moment où l'échantillon biologique de la partie a) a été obtenu, ou avec une valeur de référence,
dans lequel un niveau d'expression accru par rapport au niveau d'expression du récepteur TAS1R3 dans un échantillon biologique du même patient obtenu à un moment antérieur au moment où l'échantillon biologique de la partie a) a été obtenu, ou avec une valeur de référence, est révélateur d'une évolution clinique négative du patient.

5. Procédé pour le suivi de la réponse au traitement d'un patient souffrant de présence de tumeur, présence néoplasique ou métastatique, qui comprend les étapes suivantes :
a) à partir d'au moins un échantillon biologique isolé du patient, déterminer l'expression de TAS1R3 dans les cellules dudit échantillon biologique, et
c) comparer ledit niveau d'expression avec le niveau d'expression du récepteur TAS1R3 dans un échantillon biologique du même patient obtenu à un moment antérieur au moment où l'échantillon biologique de la partie a) a été obtenu, ou avec une valeur de référence,
dans lequel un niveau d'expression accru par rapport au niveau d'expression du récepteur TAS1R3 dans un échantillon biologique du même patient obtenu à un moment antérieur au moment où l'échantillon biologique de la partie a) a été obtenu, ou avec une valeur de référence, est révélateur d'un manque de réponse au traitement.

6. Procédé selon l'une quelconque des revendications 2 à 5, dans lequel la détermination de l'expression de TAS1R3 dans les cellules d'un échantillon biologique est réalisée avec des aptamères, des anticorps ou des fragments de ceux-ci parmi la liste consistant en Fv, Fab, Fab' et F(ab')2, scFv, ou avec des diacorps, triacorps, tétracorps et/ou des anticorps humanisés capables de se lier sélectivement au récepteur TAS1R3.

7. Procédé selon la revendication 6, dans lequel lesdits aptamères, anticorps ou fragments de ceux-ci, ou diacorps, triacorps, tétracorps et/ou anticorps humanisés, sont capables de se lier sélectivement à l'une quelconque des séquences SEQ ID NO 1 ou SEQ ID NO 2.

8. Procédé selon l'une quelconque des revendications 2 à 7, dans lequel la tumeur présente est une tumeur solide, ou dans lequel le patient a une maladie sélectionnée dans le groupe consistant en : cancer du sein, mélanome, mélanome uvéal, cancer du pancréas, cancer du poumon, cancer de la prostate, cancer de l'estomac, cancer de la tête et du cou, sarcome, glioblastome, neuroblastome, cancer du côlon et du rectum, cancer de la tête et du cou, cancer du rein et de la vessie, et hépatocarcinome.

9. Utilisation du récepteur de membrane cellulaire TAS1R3 en tant que marqueur moléculaire/biomarqueur de tumeur pour diriger des thérapies contre des cellules tumorales à travers l'utilisation de ligands du récepteur, préférablement des ligands sous forme de conjugués ou d'immunotoxines.

10. Récepteur de membrane cellulaire TAS1R3 pour son utilisation en tant que marqueur moléculaire/biomarqueur de tumeur pour le diagnostic *in vivo,* à travers l'utilisation de ligands du récepteur, préférablement des ligands sous la forme de conjugués avec des produits radiopharmaceutiques ;
dans lequel les ligands du récepteur TAS1R3 sont ces molécules capables de se lier au récepteur TAS1R3 sélectionnées de mono et disaccharides ; édulcorants artificiels tels que la sucralose ; cyclamate ; néohespéridine ; dihydrochalcone ; inhibiteurs de goût sucré tels que le lactisole ; brazzéine ; anticorps ou fragments d'anticorps.

11. Conjugué qui comprend :
a) un agent cible spécifique capable de se lier au récepteur TAS1R3, et,
b) un agent cytotoxique, un radio-isotope, une nanostructure ou une nanoémulsion ;
dans lequel l'agent cible spécifique capable de se lier au récepteur TAS1R3 est sélectionné de la liste consistant en : mono et disaccharides ; édulcorants artificiels tels que la sucralose ; cyclamate ; néohespéridine ; dihydrochalcone ; inhibiteurs de goût sucré tels que le lactisole ; brazzéine ; anticorps ou fragments d'anticorps ;
pour son utilisation en thérapie contre des cellules tumorales ou pour son utilisation comme biomarqueur de tumeur de diagnostic *in vivo.*

12. Conjugué pour son utilisation selon la revendication 10, dans lequel l'agent cible est sélectionné de la liste consistant en : aptamères, anticorps, fragments d'anticorps consistant en : Fv, Fab, Fab' et F(ab')2, scFv, ou diacorps, triacorps, tétracorps et/ou anticorps humanisés, dans lequel lesdits agents cibles sont capables de se lier sélectivement à l'une quelconque des séquences SEQ ID NO 1 ou SEQ ID NO 2.

13. Conjugué pour son utilisation selon l'une quelconque des revendications 11 à 12, dans lequel ladite utilisation est pour le traitement ou diagnostic *in vivo* d'une tumeur solide, préférablement une tumeur sélectionnée du groupe suivant : cancer du sein, mélanome, mélanome uvéal, cancer du pancréas, cancer du poumon, cancer de la prostate, cancer de l'estomac, cancer de la tête et du cou, sarcome, glioblastome, neuroblastome, cancer du côlon et du rectum, cancer de la tête et du cou, cancer du rein et de la vessie, et hépatocarcinome.
